(19) Europäisches Patentamt
European Patent Office
Office européen des brevets

(11) Publication number: 0 292 191
A1

(12) **EUROPEAN PATENT APPLICATION**

(21) Application number: 88304286.3

(22) Date of filing: 11.05.88

(51) Int. Cl.4: C07D 487/04 , A61K 31/40 , //(C07D487/04,209:00,205:00)

(30) Priority: 21.05.87 US 52307

(43) Date of publication of application: 23.11.88 Bulletin 88/47

(84) Designated Contracting States: CH DE FR GB IT LI NL

(71) Applicant: **MERCK & CO. INC.**
**126, East Lincoln Avenue P.O. Box 2000**
**Rahway New Jersey 07065-0900(US)**

(72) Inventor: **Christensen, Burton G.**
**770 Anderson Avenue Apt. 17D**
**Cliffside Park NJ 07010(US)**
Inventor: **Salzmann, Thomas N.**
**154 Meadowbrook Drive**
**North Plainfield NJ 07062(US)**
Inventor: **Schmitt, Susan M.**
**50 M Southwyck Village**
**Scotch Plains NJ 07076(US)**

(74) Representative: **Hesketh, Alan, Dr.**
**European Patent Department Merck & Co., Inc. Terlings Park Eastwick Road**
**Harlow Essex, CM20 2QR(GB)**

(54) **2-(Substituted methyl)-1-alkylcarbapenem derivatives.**

(57) This invention relates to 2-(substituted methyl)-1-alkylcarbapenem derivatives having utility as antibiotic agents.

EP 0 292 191 A1

Xerox Copy Centre

# 2-(SUBSTITUTED METHYL)-1-ALKYLCARBAPENEM DERIVATIVES

## BACKGROUND OF THE INVENTION

### (a) Field of the Invention

This invention relates to 2-(substituted methyl)-1-alkylcarbapenem derivatives of Formula I that are useful as antibiotic agents.

Thienamycin (Formula A) is a long-known broad spectrum antibacterial agent.

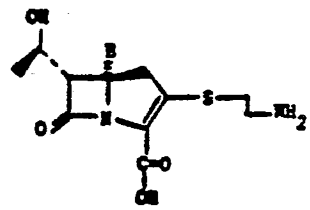

A

Subsequent discoveries include N-formimidoyl thienamycin (Formula B).

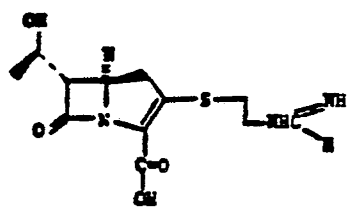

B

More recent developments include 2-(substituted thio)-1-methylcarbapenems of Formula C. Shih et al., Heterocycles, 21, 29-40 (1984).

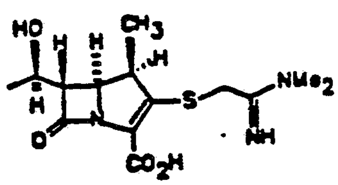

C

A continuing need exists for new antibiotics. Known antibiotics may be effective against only certain strains of microorganisms. Furthermore, and perhaps more seriously, continued widescale use can give rise to resistant strains of microorganisms against which an antibiotic was previously effective. The compounds of this invention were discovered as part of the search for new antibiotics intended to overcome such problems.

(b) Prior Art

Various 2-(substituted methyl)carbapenems have been disclosed. For example, British Patent 2,092,147 discloses compounds of Formula D

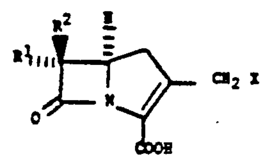

D

wherein one of $R^1$ and $R^2$ may be hydrogen and the other is hydrogen or a substituted or unsubstituted $C_1$-$C_4$ alkyl group; and X may be <u>inter alia</u> a substituted or unsubstituted $C_1$-$C_3$ alkanoyloxy group or a variously substituted thio function. Similarly, Japanese Patent Application 56-60852 discloses compounds of Formula E

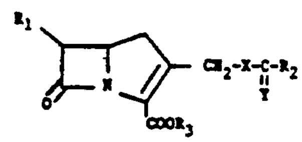

E

wherein $R^1$ may be hydrogen or alkyl (optionally substituted with hydroxy or other groups); $R_2$ may be <u>inter alia</u> amino, alkyl, or aryl; $R_3$ may be hydrogen or a physiologically labile protecting group; X may be -O- or -S-; and Y may be oxo or thioxo. Neither the British patent nor the Japanese application discloses nor suggests the 1-alkyl substitution that is a key feature of the compounds of this invention.

Various 1-substituted carbapenems have been disclosed. For example, see U.S. Patent Nos. 4,347,355; 4,312,871; 4,262,011; 4,262,010; 4,262,009; and 4,260,627. See also Shih <u>et al.</u>, <u>Heterocycles</u>, <u>21</u>, 29-40 (1984) (see Formula C, above). None of the compounds therein disclosed, however, possesses the 2-(substituted methyl) substituents of the present invention.

European Patent Application Publication No. 0184844, published June 18, 1986 (and equivalent to U.S. Patent Application Serial No. 06/681,407, filed December 13, 1984, and having the same assignee as the present application), discloses certain 2-(substituted methyl)-1-methylcarbapenems of Formula F

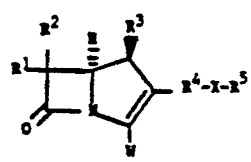

F

wherein $R^1$ and $R^2$ are independently hydrogen or alkyl optionally substituted with hydroxy or fluorine; $R^3$ is methyl; W is optionally substituted carboxy; $R^4$ may be <u>inter alia</u> $C_1$-$C_4$ alkylene; X may be <u>inter alia</u> -S-, -O-, -or -NH-; and $R^5$ may be <u>inter alia</u> $C_1$-$C_{10}$ alkyl optionally substituted with such groups as

$O\overset{O}{\overset{\parallel}{C}}R_a$, $O\overset{O}{\overset{\parallel}{C}}NR_aR_b$, $\overset{O}{\overset{\parallel}{C}}R_a$, $CONHR_a$, $CONR_aR_b$,

and the like. Unlike the compounds of the present invention, however, in no case is the group X of compound F directly attached to a carbonyl group.

SUMMARY OF THE INVENTION

This invention relates to 2-(substituted methyl)-1-alkylcarbapenems of Formula I having utility as antibiotic agents.

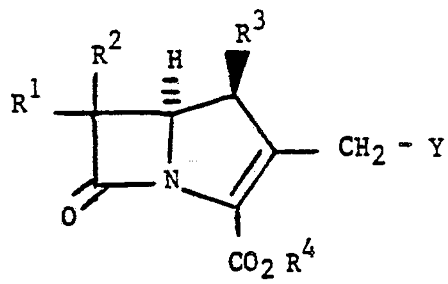

I

wherein $R^1$ and $R^2$ are independently:

a) hydrogen;

b) $C_1$-$C_4$ alkyl;

c) 1-hydroxy-substituted $C_1$-$C_4$ alkyl; or

d) fluorinated $C_1$-$C_4$ alkyl; with the proviso that at least one of $R^1$ and $R^2$ is hydrogen; $R^3$ is $C_1$-$C_4$ alkyl;

$R^4$ is:

a) hydrogen, or a pharmaceutically acceptable ester or base addition salt thereof; or

b) a removable carboxy protecting group; and Y is:

a) $OR^5$,

wherein $R^5$ is:

i) $C_2$-$C_6$ alkanoyl; or

ii) $-(C=O)NR^8R^9$,

wherein $R^8$ is:

A) hydrogen;

B) $C_1$-$C_4$ alkyl;

C) $C_1$-$C_4$ haloalkyl;

D) phenyl; or

E) $C_1$-$C_4$ alkyl substituted with

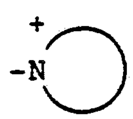

;

and

wherein $R^9$ is:

A) hydrogen; or

B) $C_1$-$C_4$ alkyl; or

wherein $R^8$ and $R^9$ taken together are $C_4$-$C_6$ alkylene;

b) $NR^6R^7$, or a pharmaceutically acceptable acid addition salt,

wherein $R^6$ is:

i) hydrogen;

ii) $C_1$-$C_4$ alkyl;

iii) $C_2$-$C_6$ alkanoyl;

iv) $-(C=O)NR^{10}R^{11}$,

wherein $R^{10}$ and $R^{11}$ are independently:

v) $-(C=NR^{13})R^{12}$,

wherein $R^{12}$ is:

A) hydrogen;

B) $C_1$-$C_4$ alkyl; or

C) $NR^{14}R^{15}$,

wherein $R^{14}$ and $R^{15}$ are independently:
I) hydrogen; or
II) $C_1$-$C_4$ alkyl; or
wherein $R^{14}$ and $R^{15}$ taken together are $C_4$-$C_6$ alkylene; or
wherein $R^{13}$ and $R^{14}$ taken together are $C_2$-$C_4$ alkylene; and
wherein $R^{13}$ is:
A) hydrogen; or
B) $C_1$-$C_4$ alkyl; and
wherein $R^7$ is:
i) hydrogen; or
ii) $C_1$-$C_4$ alkyl; or
c) $N_3$.

The term "$C_1$-$C_4$ alkyl" refers to straight or branched chain aliphatic hydrocarbon groups having from 1 to 4 carbon atoms, also referred to as lower alkyl. Examples of $C_1$-$C_4$ alkyl are methyl, ethyl, propyl, butyl, and the isomeric forms thereof.

The term "1-hydroxy substituted $C_1$-$C_4$ alkyl" refers to $C_1$-$C_4$ alkyl bearing a hydroxy group on the carbon atom most proximately attached to the $\beta$-lactam function. Examples of 1-hydroxy-substituted $C_1$-$C_4$ alkyl are hydroxymethyl, 1-hydroxyethyl, 1-hydroxypropyl, 1-hydroxybutyl, 1-hydroxy-2-methylpropyl, and their optical isomer forms.

The term "$C_1$-$C_4$ fluorinated alkyl" refers to $C_1$-$C_4$ alkyl in which one or more hydrogen atoms are replaced with fluorine atoms. Examples of $C_1$-$C_4$ fluorinated alkyl are fluoromethyl, difluoromethyl, trifluoromethyl, 1- or 2-fluoroethyl, 1,1-difluoroethyl, 2,2,2-trifluoroethyl, perfluoroethyl; other similarly monofluorinated, polyfluorinated, and perfluorinated ethyl, propyl, and butyl groups; and the isomeric forms thereof.

The term "$C_2$-$C_6$ alkanoyl" refers to straight or branched chain alkanoyl groups having from 2 to 6 carbon atoms. Examples of $C_2$-$C_6$ alkanoyl are acetyl, propanoyl, butanoyl, pentanoyl, hexanoyl, and the isomeric forms thereof.

The term "$C_1$-$C_4$ haloalkyl" refers to $C_1$-$C_4$ alkyl in which a hydrogen atom is replaced with a halogen atom. Examples of $C_1$-$C_4$ haloalkyl are halomethyl; 1- and 2-haloethyl; 1-, 2-, and 3-halopropyl; 1-, 2-, 3-, and 4-halobutyl; and the isomeric forms thereof. Examples of halo are fluoro, chloro, bromo, and iodo.

The term "$C_4$-$C_6$ alkylene" refers to aliphatic hydrocarbon chains substituted at two different carbon atoms. Examples of $C_4$-$C_6$ alkylene are $-(CH_2)_4-$, $-(CH_2)_5-$, and $-(CH_2)_6-$. As used herein, $C_4$-$C_6$ alkylene groups are taken with nitrogen atoms of $NR^8R^9$, $NR^{10}R^{11}$, and $NR^{14}R^{15}$ to form 1-azacycloalkyl groups: 1-azacyclopentyl, 1-azacyclohexyl, and 1-azacycloheptyl.

The term "$C_2$-$C_4$ alkylene" refers to aliphatic hydrocarbon chains substituted at two different carbon atoms. Examples of $C_2$-$C_4$ alkylene are $-(CH_2)_2-$, $-(CH_2)_3-$, and $-(CH_2)_4-$. As used herein, $C_2$-$C_4$ alkylene chains are taken with nitrogen atoms of $-(C=NR^{13})NR^{14}R^{15}$ to form cyclic guanidine groups.

The term "pharmaceutically acceptable acid addition salt" refers to a salt prepared by contacting a compound of Formula I with an inorganic or organic acid whose anion is generally considered suitable for human consumption. Examples of pharmaceutically acceptable acid addition salts include the acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, heptanoate, hexanoate, hydrochloride, hydrobromide, hydroiodide, 2-hydroxyethanesulfonate, lactate, malate, maleate, methanesulfonate, 2-naphthalenesulfonate, nicotinate, oxalate, pamoate, pectinate, 3-phenylpropionate, phosphate, picrate, pivalate, propionate, succinate, sulfate, tartrate, thiocyanate, tosylate, and undecanoate salts.

The term "pharmaceutically acceptable base addition salt" refers to salt prepared by contacting a compound of Formula I with a base whose cation is generally considered suitable for human consumption. Examples of pharmaceutically acceptable addition salts include lithium, sodium, potassium, magnesium, calcium, aluminum, titanium, zinc, ammonium, alkylammonium, dialkylammonium, trialkylammonium, tetraalkylammonium, diethanolamine, triethanolamine, guanidinium, lysine, benzathine, chloroprocaine, choline, meglumine, and procaine salts. It will also be noted that compounds of this invention that bear a basic nitrogen atom are potentially internal salts or zwitterions.

The term "pharmaceutically acceptable ester" refers to compounds of Formula I in which $R^4$ is any one of several groups that would be readily apparent to one skilled in the art. Examples of pharmaceutically acceptable esters include those described in detail in U.S. Pat. No. 4,309,438, Column 9, line 61, to Column 12, line 51, which is incorporated herein by reference. Included within such pharmaceutically acceptable esters are those which are hydrolyzed under physiological conditions, such as pivaloyloxymethyl, acetoxymethyl, phthalidyl, indanyl, and methoxymethyl, and those described in detail in the U.S. Pat. No. 4,479,947, which is incorporated herein by reference.

The group

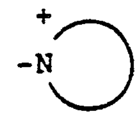

is a monocyclic heteroaromatic group having 5 or 6 ring atoms in which one or more of the carbon atoms has been replaced by a nitrogen atom and attachment of said group is by way of a nitrogen atom, and in which one or more additional carbon atoms are optionally replaced by a heteroatom selected from O and S or in which from 1 to 3 additional carbon atoms are each optionally replaced by a nitrogen atom. Useful examples of the

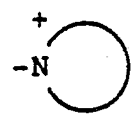

group include the following:

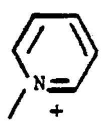
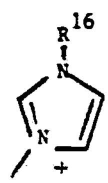
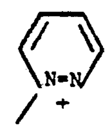

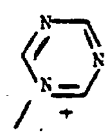
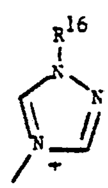
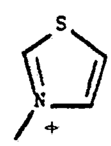

wherein $R^{16}$ is $C_1$-$C_4$ alkyl, $CH_2SO_3^-$, or $CH_2COR^{17}$ (where $R^{17}$ is hydroxyl, $NH_2$, O($C_1$-$C_4$ alkyl), or O-benzyl). The pyridinium group is generally preferred.

## DESCRIPTION OF THE INVENTION

The compounds of this invention may be prepared by the methods illustrated in the following Schemes. Unless otherwise specified, the various substituents are defined as for Formula I, above. Scheme A illustrates a method of preparing key intermediate 2-hydroxymethylcarbapenems of Formula V.

SCHEME A

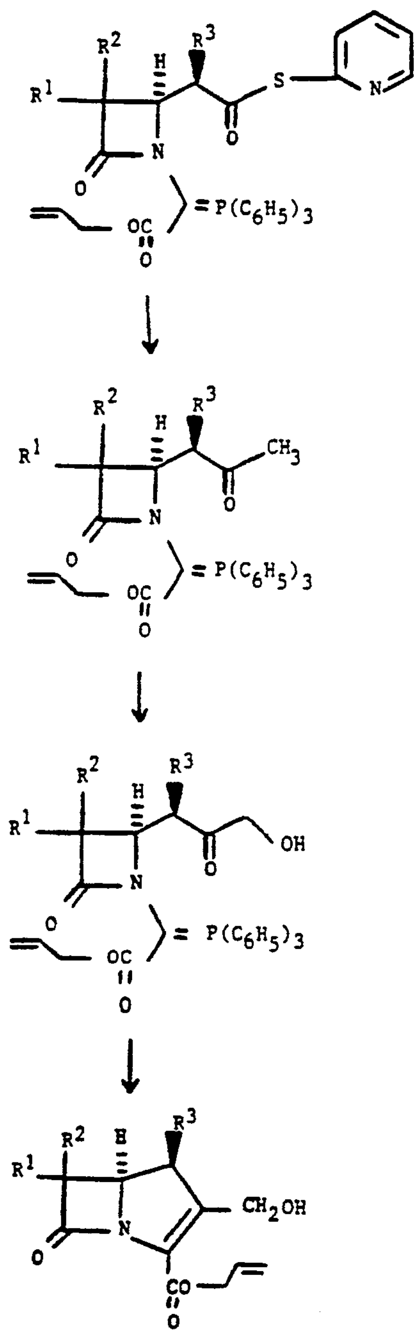

II

III

IV

V

Preparation of the thiopyridyl ester of Formula II is described by Guthikonda et al., J. Med. Chem., 30, 871-890 (1987). Compounds of Formula II are converted to corresponding methyl ketones of Formula III using suitable methods known to those skilled in the art. A preferred method employs an organometallic reagent, preferably a Grignard reagent such as methyl magnesium bromide, in a suitable organic solvent. Suitable organic solvents are organic liquids in which reactants may be dissolved or suspended but which are otherwise chemically inert. Examples of suitable organic solvents include alkanes and cycloalkanes; ethers and cyclic ethers; aromatic hydrocarbons; and other solvents known in the art. Preferred organic solvents include tetrahydrofuran and diethyl ether. The reactions are performed at temperatures below about -20° C, preferably at about -75° C to -80° C. Where $R^1$ or $R^2$ of Formula I is to be a 1-hydroxy-substituted $C_1$-$C_4$ alkyl group, the hydroxyl group is protected with a suitable protecting group, preferably a trisubstituted silyl group such as triethylsilyl or dimethyl-t-butylsilyl.

Methyl ketones of Formula III are converted to corresponding hydroxymethyl ketones of Formula IV by methods known in the art. A preferred method employs oxodiperoxymolybdenum (pyridine) (hexamethylphosphorictriamide) ("MoOPH"). See Vedejs et al., J. Org. Chem., 43, 188-196 (1978).

Preferred hydroxylation conditions involve initially forming an enolate anion by reaction of a compound of Formula III with a suitable strong base in a suitable organic solvent such as described above. Suitable strong bases include alkali metal alkoxides, such as sodium methoxide, sodium ethoxide, and potassium t-butoxide; alkali metal alkyls, such as n-butyllithium and t-butyllithium; alkali metal salts of amines, such as lithium diisopropylamide and lithium hexamethyldisilazide; and other such bases known in the art. A preferred strong base is lithium diisopropylamide. The MoOPH reagent is then added according to the general method described by Vedejs et al. The reactions are performed at temperatures below about -20° C, preferably at about -30° C to -80° C. Where $R^1$ or $R^2$ of Formula I is to be a 1-hydroxy-substituted $C_1$-$C_4$ alkyl group, the reaction employing the MoOPH reagent is most conveniently performed on compounds of Formula III in which the hydroxyl group is protected with a protecting group that can later be removed under conditions that also remove the allyl ester function. A preferred protecting group is allyloxycarbonyl. The protecting group used in the preparation of corresponding compounds of Formula III is removed using appropriate standard deprotecting procedures, for example by treatment with acid medium or fluoride ion where the protecting group is trisubstituted silyl. Reprotection with the allyloxycarbonyl group is effected using acylation methods known in the art. Preferred acylation conditions employ allyl chloroformate in a suitable organic solvent in the presence of a suitable base. Suitable organic solvents for acylation are organic liquids in which reactants may be dissolved or suspended but which do not themselves form significant quantities of byproducts by reaction with other chemical reagents or with compounds of Formula III. Examples of suitable organic solvents include alkanes and cycloalkanes; ethers and cyclic ethers; aromatic hydrocarbons; halocarbons, such as chloroform, dichloromethane, ethylene dichloride, and the like; and other solvents known in the art. Preferred organic solvents include dichloromethane. Suitable bases for acylation are chemical compounds that are sufficiently basic to prevent the reaction medium from becoming acidic but which do not themselves form significant quantities of byproducts by reaction with other chemical reagents or with compounds of Formula III. Examples of suitable bases include alkali metal bicarbonates, such as lithium, sodium, or potassium bicarbonate; alkali metal carbonates, such as lithium, sodium, or potassium carbonate; alkaline earth carbonates, such as calcium carbonate or barium carbonate; and tertiary amines, such as triethylamine, tributylamine, N-methylmorpholine, and the like; N-containing aromatic species, such as pyridine, quinoline, dimethylaminopyridine, and the like. A preferred base is 4-dimethylaminopyridine. Allyloxycarbonyl-protected compounds of Formula III are converted to corresponding compounds of Formula IV using the general methods described above.

Hydroxymethyl ketones of Formula IV are converted to 2-hydroxymethyl-1-alkylcarbapenems of Formula V by a thermally induced ring closure. Ring closure is effected, for example, by heating compounds of Formula IV in a suitable solvent, preferably a solvent heated at reflux. Suitable solvents for ring closure are liquids in which reactants may be dissolved or suspended but which themselves do not form significant quantities of byproducts by reacting with chemical reagents or products. Suitable solvents include organic liquids such as benzene, toluene, xylene, or other aromatic hydrocarbons that can be heated sufficiently to induce ring closure. A preferred solvent is toluene.

The 2-hydroxymethyl-1-alkylcarbapenems of Formula V may be deprotected to yield compounds of Formula VI (that is, Formula I in which Y is OH) or may be used to prepare the compounds of this invention, Formula I.

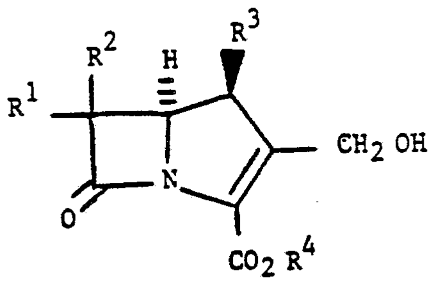

VI

Each reaction path involves removal of the allyl ester group and, where $R^1$ or $R^2$ of Formula I is to be 1-hydroxy-substituted $C_1$-$C_4$ alkyl, concomitant removal of an allyloxycarbonyl protecting group. A preferred method for removing the allyl ester and allyloxycarbonyl groups employs a palladium(O)-(trisubstituted

phosphine) complex, preferably tetrakistriphenylphosphine palladium(O), in a suitable organic solvent system containing an alkanoic acid buffer system. See Jeffrey and McCombie, J. Org. Chem., 47, 587-590 (1982). Suitable organic solvent systems are organic liquids and mixtures of organic liquids in which reactants may be dissolved but which are otherwise chemically inert. Examples of suitable organic solvent systems include alkanes and cycloalkanes; ethers and cyclic ethers; aromatic hydrocarbons; halocarbons, such as chloroform, dichloromethane, ethylene dichloride, and the like; alkanoate esters, such as ethyl acetate; other solvents known in the art; and mixtures thereof. Preferred organic solvent systems include dichloromethane-ethyl acetate mixtures. Preferred alkanoic acid buffer systems employ mixtures of 2-ethylhexanoic acid and an alkali metal 2-ethylhexanoate salt (preferably in an equimolar ratio), which affords alkali metal salts (in which $R^4$ is an alkali metal ion). Free acids and other pharmaceutically acceptable base addition salts may be prepared by any of several methods known to those in the art. Similarly, pharmaceutically acceptable esters may be prepared by any of several esterification methods known to those in the art.

Scheme B illustrates general methods for preparing 2-(substituted methyl)-1-alkylcarbapenems, Formula I, of this invention.

SCHEME B

V

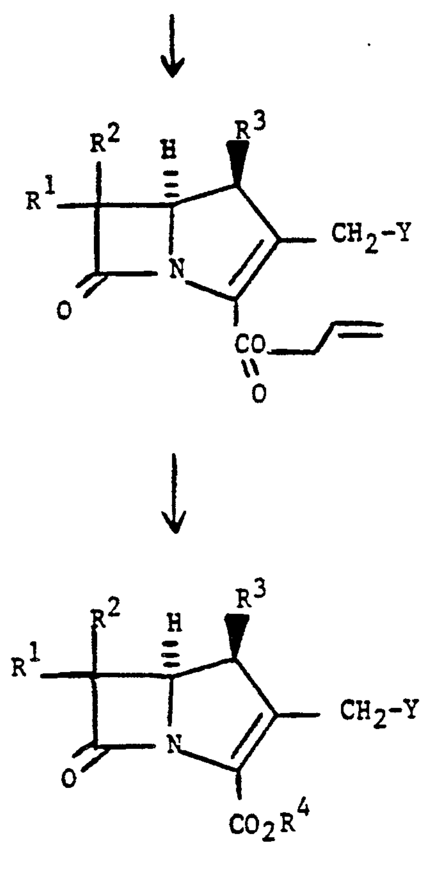

VII

I

A 2-hydroxymethyl-1-alkylcarbapenem of Formula V may be converted to compounds of Formula VII by any of several methods known in the art. The method used will vary according to the particular group Y that is to be introduced. For example, where Y is to be $OR^5$, the desired compound of Formula I is prepared by acylating the 2-hydroxymethyl-1-alkylcarbapenem of Formula V. A preferred acylation method for preparing compounds of Formula VII in which $R^5$ is $C_2$-$C_6$ alkanoyl uses diethylazodicarboxylate and triphenylphosphine to activate the corresponding $C_2$-$C_6$ alkanoic acid. A preferred acylation method for preparing compounds of Formula VII in which $R^5$ is $-(C=O)NR^8R^9$ uses an isocyanate reagent (giving a compound in which one of $R^8$ or $R^9$ is hydrogen) or involves an initial reaction with phosgene followed by reaction with

an amine of the formula $HNR^8R^9$. Depending on the nature of $R^8$ and $R^9$, the resultant carbamate may be further modified using methods known in the art.

Where Y of Formula VII is to be $NR^6R^7$, a preferred method of converting the 2-hydroxymethyl group of a compound of Formula V to corresponding 2-aminomethyl compounds involves forming the corresponding 2-azidomethyl compounds (that is, Formula VII in which Y is $N_3$). A preferred method for preparing the 2-azidomethyl compounds employs a mixture of diethylazodicarboxylate, hydrazoic acid, and triphenylphosphine in a suitable solvent, preferably benzene or toluene. The 2-azidomethyl compounds may be deprotected (as above) to yield the 2-azidomethyl-1-alkylcarbapenems of this invention (Formula I in which Y is $N_3$) or may be used to prepare the 2-aminomethyl-1-alkylcarbapenems of this invention (Formula I in which Y is $NR^6R^7$). Reduction of the azido function to the amino function may be effected using any several of the methods known in the art. A preferred method involves a reaction of the 2-azidomethyl-1-alkylcarbapenems with a mixture of triphenylphosphine and allyl chloroformate in a suitable solvent system. Although many solvent systems are suitable, a preferred solvent system is a heterogeneous mixture of water containing a bicarbonate salt and a water-immiscible organic solvent such as diethyl ether. The amino compound is isolated as the N-allyloxycarbonylamino derivative. The allyl deprotection method described above produces 2-aminomethyl-1-alkylcarbapenems of Formula I in which Y is $NH_2$. The amino group may then be further derivatized using methods known in the art. Where further derivatization would be hindered by the presence of other deprotected groups, the amino group could be trapped during reduction by acylating reagents other than allyl chloroformate, such that the amino group could be deprotected without disturbing the allyl-containing functions. Such procedures could readily be devised by those skilled in the art.

Where Y of Formula VII is to be $NR^6R^7$, an alternative method involves aminating the methyl ketone group of a compound of Formula III (see Scheme A) before ring closure. Scheme C illustrates an alternative preferred method for preparing 2-aminomethyl compounds of Formula XI (that is, Formula I in which Y is $NH_2$ and $R^4$ is hydrogen).

## SCHEME C

III

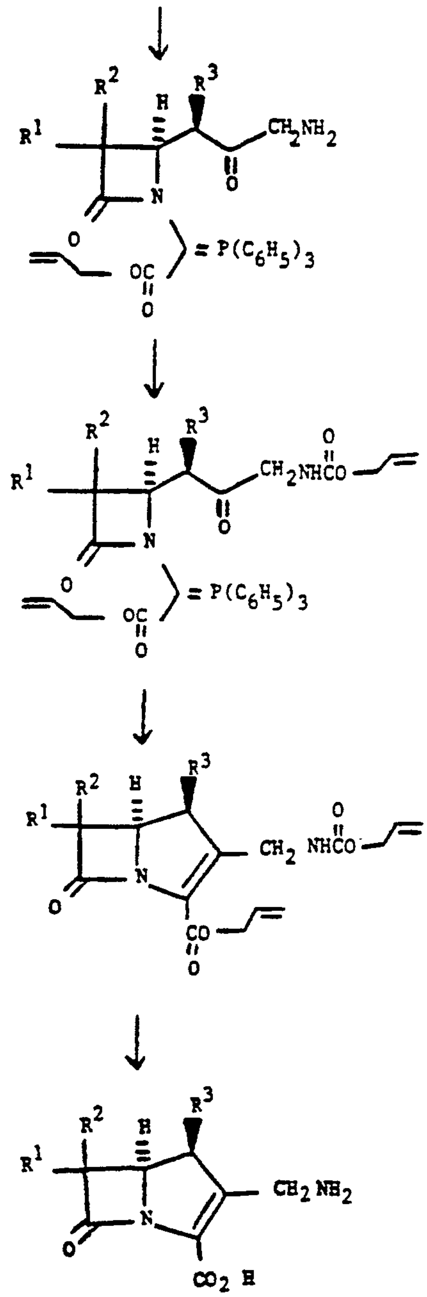

VIII

IX

X

XI

Compounds of Formula III are aminated using methods known in the art to form corresponding aminomethyl compounds of Formula VIII. A preferred method involves generating an anionic intermediate by adding a suitable strong base to a mixture of a compound III in a suitable organic solvent, followed by addition of a suitable O-substituted hydroxylamine derivative. Suitable strong bases and organic solvents include those described above for the hydroxylation of a compound of Formula III (illustrated in Scheme A) Suitable O-substituted hydroxylamines are hydroxylamine derivatives which the O-substituent forms a stable leaving group that does not form significant reaction byproducts after being released during the amination. Examples of suitable O-substituted hydroxylamines include O-(2,4,6-triisopropylbenzenesulfonyl)-hydroxylamine, O-mesitylenesulfonylhydroxylamine, O-(diphenylphosphinyl)hydroxylamine, and other such hydroxylamine derivatives known in the art. Preferred amination conditions use lithium diisopropylamide in

tetrahydrofuran to generate the anionic intermediate and O-(2,4,6-triisopropylbenzenesulfonyl)hydroxylamine to complete the amination.

The aminomethyl group is protected before ring closure, preferably with an allyloxycarbonyl group as shown in Formula IX. The protecting group is attached using methods well known in the art, preferably by reaction of compound VIII with allyl chloroformate.

Protected aminomethyl compounds of Formula IX are converted to corresponding 2-aminomethyl-1-alkylcarbapenems of Formula X by a thermally induced ring closure. Preferred ring closure methods are the same as those used to convert compounds of Formula IV to compounds of Formula V (described above and illustrated in Scheme A). Protecting groups are removed using methods described above to yield compounds of Formula XI of this invention. As discussed above, the amino group and carboxylic acid group may be further derivatized using methods known in the art to form other compounds within the scope of this invention.

The preferred embodiments of this invention include compounds of the following general structure:

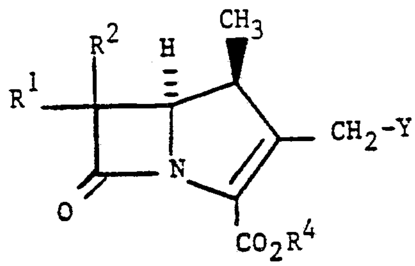

XII

wherein one of $R^1$ or $R^2$ is hydrogen and the other of $R^1$ or $R^2$ is 1-hydroxy-substituted $C_1$-$C_4$ alkyl; $R^4$ is hydrogen or a pharmaceutically acceptable base addition salt thereof; and Y is $OR^5$ (where $R^5$ is defined as above), $NR^6R^7$ (where $R^6$ and $R^7$ are independently hydrogen or $C_1$-$C_4$ alkyl), or $N_3$.

The most preferred embodiments of this invention include compounds of the following general structure:

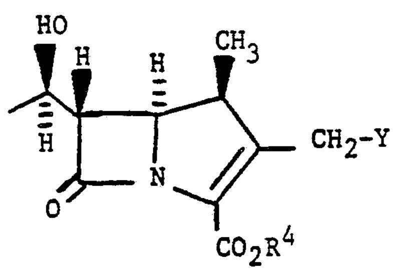

XIII

wherein $R^4$ is hydrogen or a pharmaceutically acceptable base addition salt thereof; and Y is $OR^5$ (where $R^5$ is defined as above), $NH_2$, or $N_3$.

The compounds of this invention exhibit antibacterial activity, as indicated by an agar disc-diffusion assay in which thienamycin and other carbapenems exhibit such activity. The antibacterial activities of the compounds of this invention illustrated in the examples were determined using the following method.

RELATIVE ANTIBACTERIAL ACTIVITY

Antibacterial activities were determined by the disc-diffusion assay described by Bauer et al., Amer. J. Clin. Pathol., 45, 493 (1966), except that an agar thickness of 0.2 cm was used. Thienamycin or imipenem was used as the internal standard. For maximum reproducibility, the inner, completely clear region of the zone of inhibition was measured. Inhibiting concentration at the edge of the zone was computed for each test compound using equation (3) (rearranged) reported by Humphrey and Lightbown in J. Gen. Microbiol., 7, 129 (1952), which takes into account differing molecular weights and resulting diffusion constants. Ratios of the geometric means of the inhibitory concentrations to that of thienamycin were determined for each test compound.

The compounds of the present invention are valuable antibacterial agents active against various Gram-positive and Gram-negative bacteria and accordingly find utility in human and veterinary medicine. Representative pathogens which are sensitive to the antibacterial agents of the present invention include various species of the following: Staphylococcus Enterococcus, Escherichia, Klebsiella, Enterobacter, Bacillus, Salmonella, Pseudomonas, Serratia and Proteus. The antibacterials of this invention are not limited to utility as medicaments; they may be used in all manner of industry, for example, as additives to animal feed, as preservatives of food, as disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in white water of paper mills to inhibit the growth of harmful bacteria.

Regardless of the route of administration selected, the compounds of the present invention are formulated into pharmaceutically acceptable dosage forms by conventional methods known to those skilled in the art. The compounds may also be formulated using pharmaceutically acceptable acid or base addition salts where appropriate. Moreover, the compounds or their salts may be used in a suitable hydrated form. The compounds of this invention may be used in any of a variety of pharmaceutical preparations. The compounds may be administered in such oral dosage forms as tablets, capsules, pills, powders, granules, elixirs, or syrups. The compounds may also be administered intravascularly, intraperitoneally, subcutaneously, intramuscularly, or topically using forms known to the pharmaceutical art.

Compositions for injection, a preferred route of delivery, may be prepared in unit dosage form or in multidose containers. The compositions may take such forms as suspensions, solutions, or emulsions in oily or aqueous vehicles, and may contain formulatory agents. Alternatively, the active ingredient may be in powder form for reconstitution at the time of delivery with a suitable vehicle, such as sterile water. Topical applications may be formulated in hydrophobic or hydrophilic bases as ointments, creams, lotions, paints, or powders.

For the orally administered pharmaceutical compositions and methods of the present invention, the foregoing active ingredients will typically be administered in admixture with suitable pharmaceutical diluents, excipients, or carriers (collectively referred to herein as "carrier" materials) suitably selected with respect to the intended form of administration, that is, oral tablets, capsules, elixirs, syrups, and the like, and consistent with conventional pharmaceutical practices. For example, for oral administration in the form of tablets or capsules, the active drug components may be combined with any oral non-toxic pharmaceutically acceptable inert carrier such as lactose, starch, sucrose, cellulose, magnesium stearate, dicalcium phosphate, calcium sulfate, mannitol, and the like, or various combinations thereof; for oral administration in liquid form, the active drug components may be combined with any oral non-toxic pharmaceutically acceptable inert carrier such as water, saline, ethanol, polyethylene glycol, propylene glycol, corn oil, cottonseed oil, peanut oil, sesame oil, benzyl alcohol, various buffers, and the like, or various combinations thereof. Moreover, when desired or necessary, suitable binders, lubricants, disintegrating agents, and coloring agents can also be incorporated in the mixture. Suitable binders include starch, gelatin, natural sugars, corn sweetners, natural and synthetic gums such as acacia, sodium alginate, carboxymethylcellulose, polyethylene glycol, and waxes, or combinations thereof. Lubricants for use in these dosage forms include boric acid, sodium benzoate, sodium acetate, sodium chloride, and the like, or combinations thereof. Disintegrators include without limitation, starch, methylcellulose, agar, bentonite, guar gum, and the like, or combinations thereof. Sweetening and flavoring agents and preservatives can also be included where appropriate.

The dosage to be administered depends to a large extent upon the condition and size of the subject being treated as well as the route and frequency of administration, the parenteral route by injection being preferred for generalized infections. Such matters, however, are left to the routine discretion of the therapist according to principles of treatment well known in the antibacterial art. In general, a daily dosage consists of from about 5 to about 50 mg of active ingredient per kg of body weight of the subject in one or more treatments per day. A preferred daily dosage for adult humans lies in the range of from about 5 to 30 mg of active ingredient per kg of body weight. Another factor influencing the precise dosage regimen, apart from the nature of the infection and peculiar identity of the individual being treated, is the molecular weight of the chosen species of this invention.

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound of Formula I in sterile water solution or in the form of a soluble

powder intended for such a solution.

The preferred method of administration of the antibacterial agent of Formula I is parenteral by i.v. infusion, i.v. bolus, or i.m. injection.

For adults, 5-30 mg of Formula I antibacterial per kg of body weight given 2, 3, or 4 times per day is preferred. Preferred dosage is 250 mg to 1000 mg of the antibacterial agent of Formula I given two (b.i.d.), three (t.i.d.), or four (q.i.d.) times per day. More specifically, for mild infections, particularly of the urinary tract, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 mg t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibacterial compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibacterial agent. Inhibitors of DHP and their use with carbapenem antibacterial agents are disclosed in the prior art. See European Patent Applications No. 79102616.4 filed July 24, 1979 (Publication No. 0 010 573); 79102615.6, filed July 24, 1979 (Publication No. 0 007 614); and 82107174.3, filed August 9, 1982 (Publication No. 0 072 014).

The compounds of the present invention may, where DHP inhibition is desired or necessary, be combined or used with the appropriate DHP inhibitor as described in the aforesaid patents and published application. Thus, to the extent that the cited European patent applications define the procedure for determining DHP susceptibility of the present carbapenems and disclose suitable inhibitors, combination compositions and methods of treatment, they are incorporated herein by reference. A preferred weight ratio of Formula I compound to DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and their use are further embodiments of the present invention.

The following examples further illustrate details for the preparation of the compounds of this invention. The invention, which is set forth in the foregoing disclosure, is not to be construed or limited either in spirit or in scope by these examples. Those skilled in the art will readily understand that known variations of the conditions and processes of the following preparative procedures can be used to prepare these compounds. All temperatures are degrees Celsius unless otherwise noted.

## DESCRIPTION OF THE PREFERRED EMBODIMENTS

### Example 1

Potassium (1S,1′R,5R,6S)-2-hydroxymethyl-6-(1′-hydroxyethyl)-1-methylcarbapen-2-em-3-carboxylate

Step A:

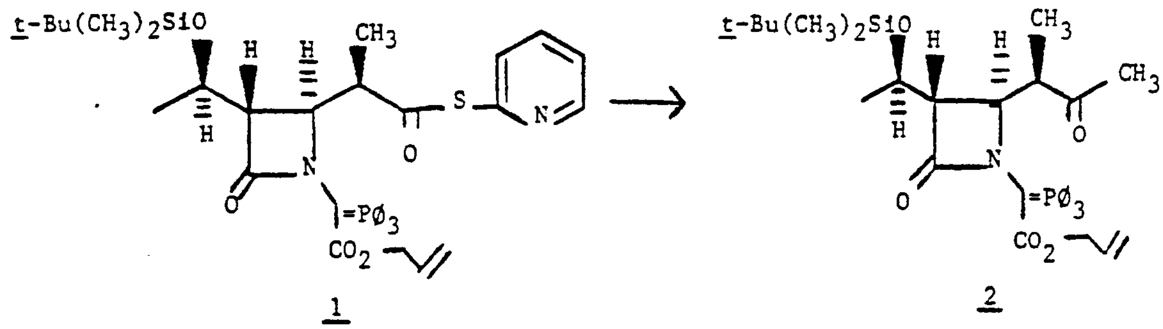

1 2

A commercial sample of 3M methyl magnesium bromide in diethyl ether (690 µl, 2.07 mmol) is added dropwise to a stirred solution of thiopyridyl ester 1 (1.06 g, 1.41 mmol) in anhydrous tetrahydrofuran (25 ml) at -78° under nitrogen. After 5 minutes an additional amount of methyl magnesium bromide (140 µl, 0.42 mmol) is added and stirring is continued at -78° for 10 minutes. The reaction mixture is then added to a saturated ammonium chloride solution (35 ml), water (10 ml), and ethyl acetate (40 ml). After phase separation the aqueous layer is extracted with an additional amount of ethyl acetate. The combined organic layers are washed sequentially with cold 1N hydrochloric acid (35 ml), cold 10% sodium bicarbonate (35 ml), and brine (35 ml). After drying over magnesium sulfate, the organic layer is concentrated in vacuo to a foam. Chromatography on silica gel (gradient elution with 0-10% ethyl acetate in methylene chloride) affords the methyl ketone (ca. 90% pure) (800mg). Preparative thin layer chromatography (TLC) on silica gel (eluting with 1:1 ethyl acetate-hexane and extracting with 1:1 ethyl acetate-methylene chloride) provides the pure methyl ketone 2 (650 mg, 70% yield). Structure assignment is consistent with the nmr and infrared spectra and mass spectrometry:

200 MHz nmr ($CDCl_3$) (selected absorbances): δ (ppm) 0.8 ($SiC(CH_3)_3$); 2.22 ($CH_3C=O$); 6.0 (m, $CH_2C\underline{H}=CH_2$); 7.4-8.0 (aromatic protons).

IR ($CH_2Cl_2$): 1750, 1720, 1650, 1625cm$^{-1}$.

MS(FAB): 658(M+1), 600 (M-t-butyl).

Step B:

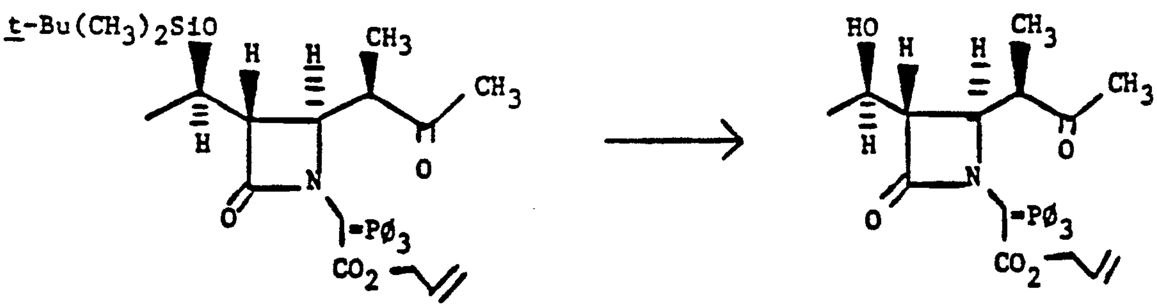

2 3

The methyl ketone 2 (38 mg, 0.058 mmol) is stirred under nitrogen at ambient temperature in 0.2 M hydrochloric acid in 9:1 methanol-water (2.4 ml) for 6 hours. After the addition of 1M dipotassium hydrogen phosphate (1.2 ml), 1M potassium dihydrogen phosphate (0.7 ml), water (5 ml), and ethyl acetate (5 ml) and phase separation, the aqueous phase was again extracted with ethyl acetate. The combined organic layers are washed with brine, dried, filtered, and concentrated in vacuo to give crude 3. Preparative TLC (eluting with ethyl acetate) provides the desilylated compound 3 (26 mg, 84%). Structure assignment is consistent

with the nmr and infrared spectra and mass spectrometry:

200MHz nmr ($CDCl_3$), (selected absorbances): δ (ppm) 1.01 (d, $C\underline{H}_3CHOH$); 1.53 (d, $C\underline{H}_3CHC=O$); 2.19 (s, $CH_3C=O$); 6.0 (m, $CH_2C\underline{H}=CH_2$); 7.4-8.0 (aromatic protons).

IR ($CH_2Cl_2$) 3550, 1745, 1700, 1640, 1620cm$^{-1}$

MS(FAB): 544(M+1), 262 ($(C_6H_5)_3P$), 185

Step C:

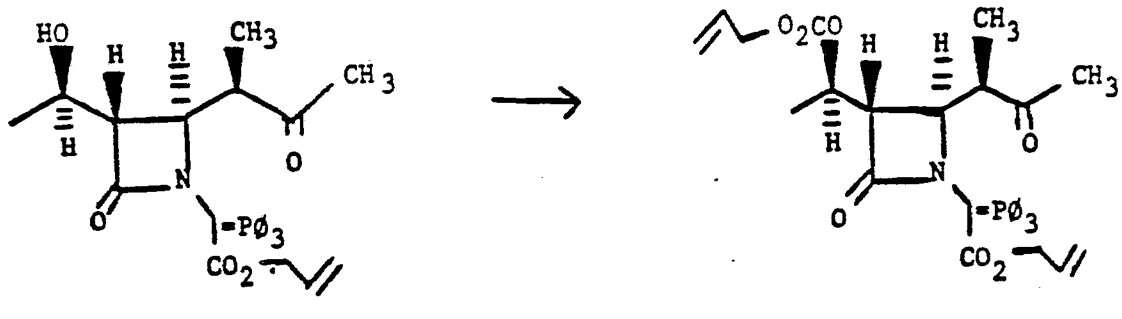

3

4

4-Dimethylaminopyridine (DMAP) (19 mg, 0.15 mmol) and allyl chloroformate (16 µl, 0.15 mmol) are added to a stirred solution of 3 (66 mg, 0.12 mmol) in dichloromethane at 0° C under nitrogen. After 10 minutes at 0° C followed by 50 minutes at ambient temperatures, equivalent amounts of DMAP and allyl chloroformate are again added. After 2.5 hours at ambient temperature equivalent amounts of DMAP and allyl chloroformate are added for a third time. After 2 hours the reaction is diluted with dichloromethane and extracted with 1M dipotassium hydrogen phosphate (1.5 ml) and 1M potassium dihydrogen phosphate (1 ml). The aqueous layer is again extracted with dichloromethane. The combined organic layers are washed with brine, dried, filtered, and concentrated in vacuo to provide the crude carbonate ester 4. Preparative TLC (eluting with 1:1 ethyl acetate-hexane) affords pure 4 (61 mg, 80%). Structure assignment is consistent with the nmr and infrared spectra and mass spectrometry:

200MHz nmr ($CDCl_3$) (selected absorbances): δ (ppm) 1.07 (d, $C\underline{H}_3CHO$); 1.38 (d, $C\underline{H}_3CHC=0$); 2.12 ($CH_3C=0$); 5.94 (m, $CH_2C\underline{H}=CH_2$); 7.4-8.0 (aromatic protons).

IR ($CH_2Cl_2$): 1750, 1725, 1650, 1625cm$^{-1}$

MS(FAB): 628(M+1), 570(M-O-allyl), 262($(C_6H_5)_3P$)

Step D:

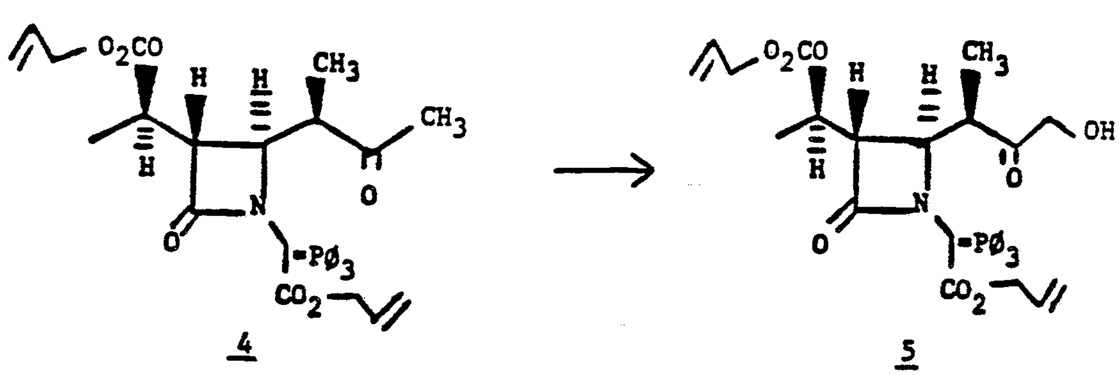

4

5

To diisopropylamine (264 µl, 1.9 mmol) in anhydrous tetrahydrofuran (9 ml) at 0° C under nitrogen is added 1.6 M butyllithium in hexane (1.2 ml, 1.9 mmol). After a few minutes, 6.5 ml of the above lithium diisopropylamide (LDA) solution (1.2 mmol) is added to a solution of 4 (591 mg, 0.94 mmol) in

tetrahydrofuran (21 ml) at -78° C. After 30 seconds oxodiperoxymolybdenum (pyridine) (hexamethylphosphoric triamide) (MoOPH) (622 mg, 1.43 mmol) is added, the reaction mixture is warmed to -30°, and stirring under nitrogen is continued for 1 hour. The reaction mixture is then poured into concentrated sodium sulfite (10 ml), 1M potassium dihydrogen phosphate (4.5 ml), water (36 ml), and ethyl acetate (55 ml) and stirred for 5 minutes. The layers are separated and the aqueous layer is extracted again with ethyl acetate. The combined organic layers are extracted with brine, dried over magnesium sulfate, filtered, and concentrated in vacuo to give the crude hydroxymethyl ketone 5. Preparative TLC (eluting with 1:1 ethyl acetate-dichloromethane) affords 5 (348 mg, 58%) as a white foam. Structure assignment is consistent with the nmr and infrared spectra and mass spectrometry:

300MHz nmr ($CDCl_3$) (selected absorbances): δ (ppm) 1.08 (d, $C\underline{H}_3CHO$); 1.39 (d, $C\underline{H}_3CHC=O$); 4.28 ($C\underline{H}_2OH$); 5.93 (m, $CH_2C\underline{H}=CH_2$); 7.4-7.9 (aromatic protons).

IR ($CH_2Cl_2$): 3540, 1740, 1640, 1620cm$^{-1}$

MS(FAB): 644(M+1), 262($(C_6H_5)_3P$)

Step E:

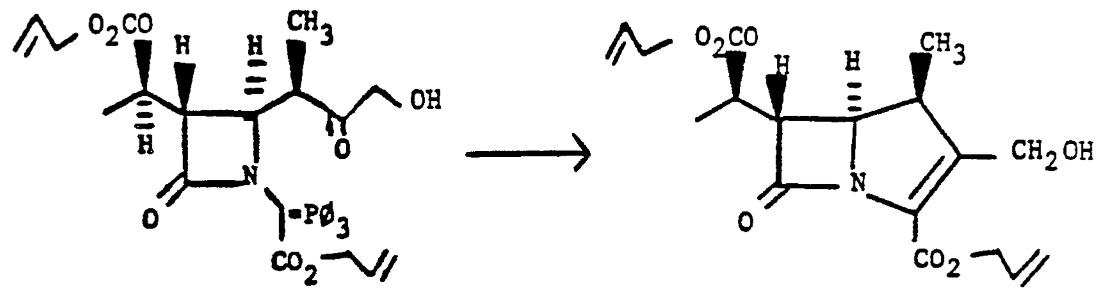

5 6

A solution of 5 (338 mg, 0.53 mmol) in toluene (35ml) is heated at reflux under nitrogen for 1 hour. After concentration in vacuo, the crude material is purified by preparative TLC (eluting with 1:1 ethyl acetate-dichloromethane) to give 6 (145 mg, 76%) as a colorless oil. Structure assignment is consistent with the nmr and infrared spectra and mass spectrometry:

300MHz nmr ($CDCl_3$) δ (ppm) 1.20 & 1.46 (two d's $C\underline{H}_3CH$ and $C\underline{H}_3CHO$); 3.14 (t, J=6Hz, OH); 3.23 (m, $H_1$); 4.18 (dd, J=3 & 8Hz, $H_6$); 4.37 (dd, J=15 & 6Hz, $C\underline{H}_2OH$); 4.50 (dd, J=15 & 6Hz, $C\underline{H}_2OH$); 4.62 & 4.77 (center of m's for two $C\underline{H}_2CH=CH_2$); 5.13 (m, $H_8$); 5.26-5.48 (m, two $CH_2CH=C\underline{H}_2$); 5.94 (m, two $CH_2C\underline{H}=CH_2$).

IR ($CH_2Cl_2$): 3560, 1775, 1740, 1720(sh)cm$^{-1}$

MS(FAB): 366 (M+1).

Step F:

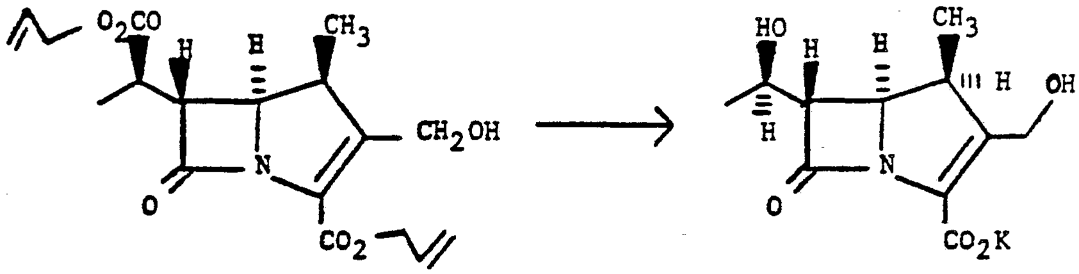

6 7

A mixture of 6 (19 mg, 0.052 mmol), triphenyl phosphine (4.0 mg, 0.0153 mmol), tetrakistriphenylphosphine palladium (4.8 mg, 0.0042 mmol), potassium 2-ethylhexanoate (0.110 ml of 0.5 M solution in ethyl acetate, 0.055 mmol), and 2-ethylhexanoic acid (0.055 mmol) in ethyl acetate (1 ml) and dichloromethane (1 ml) is stirred at ambient temperature for 2 hours. The reaction mixture is then concentrated in vacuo, and the residue is washed with diethyl ether (3x1 ml). The residual white solid is dried in vacuo, and then dissolved in water (0.3 ml) and 4% ethanol in water (0.3 ml). The resulting solution is chromatographed on two 500μ RPS-F plates (eluting with 4% ethanol in water in a cold room). The main U.V. active band is scraped off the plate and extracted with 4:1 acetonitrile-water (30 ml). The resulting extract is concentrated under high vacuum to a volume of about 6 ml. This solution is then washed with hexanes (2x2 ml), filtered through a Gelman disk filter, and concentrated to a smaller volume. Lyophilization of the concentrated solution at 0° C yields the title compound 7 (9.6 mg, 66%).

Structure assignment is consistent with the nmr and ultraviolet spectra:

200MHz nmr ($D_2O$ with no internal standard, HDO at 4.80): δ (ppm) 1.10 & 1.27 (two d's, $C\underline{H}_3CH$ and $C\underline{H}_3CHOH$); 3.26 (m, $H_1$); 3.40 (dd, J=3 & 6Hz, $H_6$); 4.21 (m, $H_5$ & $CH_3C\underline{H}O$); 4.26 (d, J=14Hz, $C\underline{H}_2OH$); 4.64 (d, J=14Hz, $C\underline{H}_2OH$).

UV ($H_2O$): λ max 268 nm ($NH_2OH$ extinguishable)

Example 2

Potassium (1S,1′R,5R,6S)-2-acetoxymethyl-6-(1′-hydroxyethyl)-1-methylcarbapen-2-em-3-carboxylate

Step A:

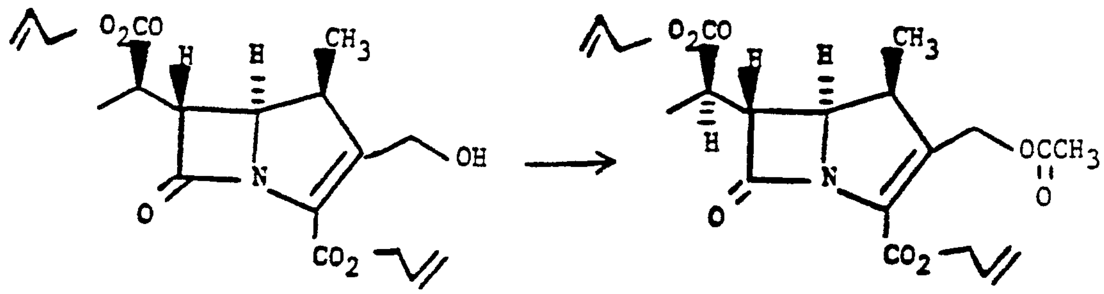

6 8

Diethylazodicarboxylate (12 µl, 0.08 mmol), acetic acid (4.5 µl, 0.08 mmol), and triphenylphosphine (21 mg, 0.08 mmol) are added to a stirred solution of 6 (25 mg, 0.07 mmol) in diethyl ether (0.7 ml) at ambient temperature under nitrogen. After 5 minutes, 0.5M pH7 phosphate buffer (1 ml) and additional diethyl ether is added. The layers are separated and the aqueous layer is again extracted with diethyl ether. The combined organic layers are washed with brine, dried over magnesium sulfate, filtered, and concentrated *in vacuo* to give crude 8. Preparative TLC (eluting with 1:1 ethyl acetate-hexane) provides pure 8 (22 mg, 75%). Structure assignment is consistent with the nmr, infrared, and ultraviolet spectra:

200MHz nmr ($CDCl_3$): δ (ppm) 1.19 and 1.47 (two d's, $C\underline{H}_3CH$ and $C\underline{H}_3CHO$); 2.10 (s, Ac); 3.29(m, $H_1$); 3.45 (dd, J=3 & 8Hz, $H_6$); 4.24 (dd, J=3 & 10Hz, $H_5$); 4.60-4.92 (m's, $CO_2C\underline{H}_2$); 4.87(d, J=15 Hz, $C\underline{H}_2OAc$); 5.16 (m, $CH_3C\underline{H}O$); 5.26-5.54 (m, $CH=C\underline{H}_2$); 5.44 (d, J=15 Hz, $C\underline{H}_2OAc$); 5.97 (m, $C\underline{H}=CH_2$)

IR ($CH_2Cl_2$): 1790, 1755cm$^{-1}$

UV (dioxane): λ max 288 nm

Step B:

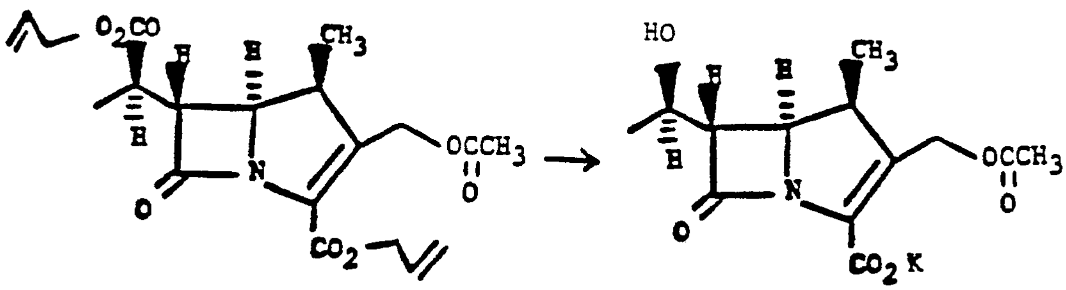

8 9

Compound 8 (22 mg, 0.054 mmol) is deblocked as in Example 1-Step F. The reaction mixture is concentrated *in vacuo*. The residue is dissolved in dichloromethane (0.8 ml) and is chromatographed on two 500µ RPS-F plates (eluting with 5% ethanol in water in a cold room). Work-up of the plates, followed by lyophilization, as described in Example 1-Step F, provides the title compound 9 (7.9 mg, 45%). Structure assignment is consistent with the nmr and ultraviolet spectra:

200MHz nmr ($D_2O$): δ(ppm) 1.10 and 1.25 (two d's, $C\underline{H}_3CH$ and $C\underline{H}_3CHO$); 2.08 (s, Ac); 3.24 (m, $H_1$); 3.42 (dd, J=2 & 6 Hz, $H_6$); 4.19 (m, $H_5$ and $CH_3C\underline{H}O$); 4.72 (part of d, 2nd part hidden under HDO, $C\underline{H}_2OAc$); 4.80 (HDO); 5.30 (d, J=12 Hz, $CH_2OAc$).

UV ($H_2O$): λ max 267 nm ($NH_2OH$ extinguishable)

Example 3

Potassium (1S,1′R,5R,6S)-2-azidomethyl-6-(1′-hydroxyethyl)-1-methylcarbapen-2-em-3-carboxylate

Step A:

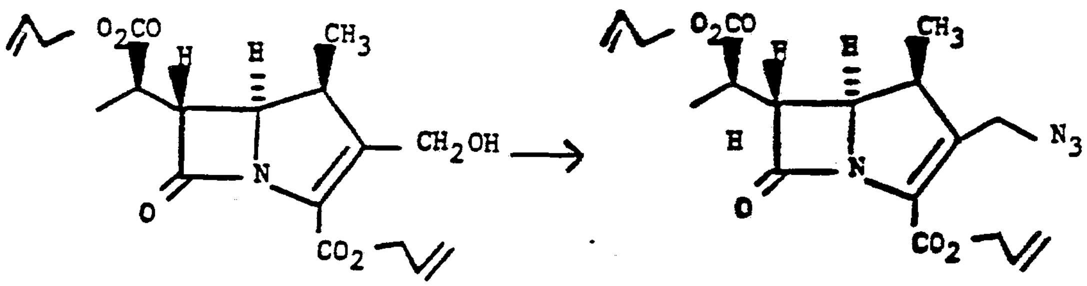

6 10

Diethylazodicarboxylate (8.5 µl, 0.054 mmol), 1.65M hydrazoic acid in benzene (33 µl, 0.054 mmol), and triphenylphosphine (14.2 mg, 0.054 mmol) are added to a stirred solution of 6 (18 mg, 0.049 mmol) in diethyl ether (0.5 ml) at ambient temperature under nitrogen. After 5 minutes 0.5M pH7 phosphate buffer (1 ml) and additional diethyl ether is added. The layers are separated and the aqueous layer is again extracted with ether. The combined organic layers are washed with brine, dried over magnesium sulfate, filtered and concentrated in vacuo to give the crude azido compound 10. Preparative TLC (eluting with 1:3 ethyl acetate-hexane) provides pure 10 (14 mg, 73%). Structure assignment is consistent with the nmr and infrared spectra:

200MHz nmr ($CDCl_3$): δ (ppm) 1.20 and 1.47 (two d's, $CH_3CH$ and $CH_3CHO$); 3.34 (m, $H_1$); 3.47 (dd, J=3 and 8 Hz, $H_6$), 4.01 (d, J=14 Hz, $CH_2N_3$); 4.28 (dd, J=3 and 10 Hz, $H_5$); 4.64-4.92 (m, $CO_2CH_2$'s); 4.85 (d, J=14 Hz, $CH_2N_3$); 5.17 (m, $CH_3CHO$); 5.26-5.54 (m, $CH=CH_2$'s); 5.98 (m, $CH=CH_2$'s).

IR ($CH_2Cl_2$): 2140, 1790, 1750, 1730$cm^{-1}$

Step B:

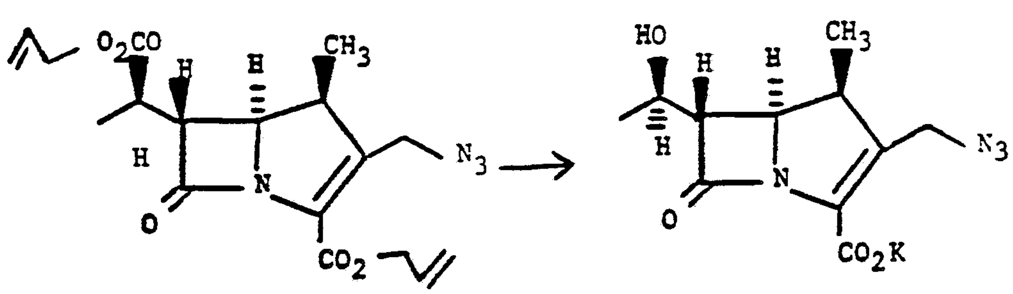

10 11

Compound 10 (56 mg, 0.14 mmol) is deblocked as in Example 1-Step F. The reaction mixture is concentrated in vacuo. The residue is dissolved in dichloromethane (1ml) and is chromatographed on two 1000µ RPS-F plates (eluting with 10% ethanol in water in a cold room). Work-up of the plates, followed by

lyophilization, as described in Example 1-Step F, provides the title compound 11 (21 mg, 48%). Structure assignment is consistent with the nmr and ultraviolet spectra:

200MHz nmr ($D_2O$): $\delta$ (ppm) 1.17 and 1.32 (two d's, $CH_3CH$ and $CH_3CHO$); 3.31 (m, $H_1$); 3.48 (dd, J=2 and 6 Hz, $H_6$); 4.02 (d, J=14 Hz, $CH_2N_3$); 4.26 (m, $H_5$ and $CH_3CHO$); 4.64 (d, J=14 Hz, $CH_2N_3$); 4.80 (HDO).

UV ($H_2O$): $\lambda$ max 269 nm

## Example 4

Potassium (1S,1′R,5R,6S)-2-aminocarbonyloxy methyl-6-(1′-hydroxyethyl)-1-methylcarbapen-2-em-3-carboxylate

### Step A:

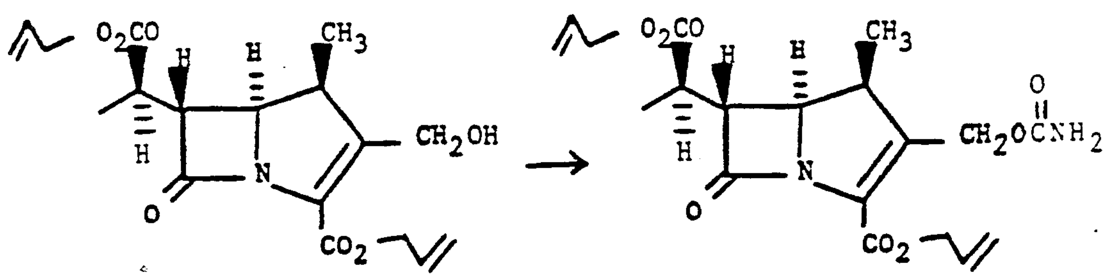

6

12

A solution of trichloroacetylisocyanate (0.073 mmol) in carbon tetrachloride (0.5 ml) is added to 6 (25 mg, 0.068 mmol) in carbon tetrachloride (2 ml). The reaction is stirred under nitrogen at ambient temperature for 1 hour and then concentrated in vacuo. The residue is dissolved in methanol (1.1 ml) and silica gel (280 mg) is added. The mixture is stirred at 35° C for 2 hours and then filtered, washing the silica gel well with 10% methanol-dichoromethane (5x2 ml) and then 50% ethyl acetate-dichloromethane (2x5 ml). The combined filtrate and washings are concentrated in vacuo, redissolved in dichloromethane, dried over magnesium sulfate, filtered, and concentrated in vacuo to provide the crude carbamate 12. Preparative TLC (eluting with 20% ethyl acetate-dichloromethane) provides pure 12 (24 mg, 85%). Structure assignment is consistent with the nmr and infrared spectra:

200MHz nmr ($CDCl_3$): $\delta$ (ppm) 1.20 and 1.47 (two d's, $CH_3CH$ and $CHCHO$); 3.30 (m, $H_1$); 3.45 (dd, J=3 & 8 Hz, $H_6$); 4.23 (dd, J=3 & 10 Hz, $H_5$); 4.60-4.94 (m, $CO_2CH_2$'s); 4.88 (d, J=14 Hz, $CH_2O$); 5.14 (m, $CH_3CHO$); 5.26-5.52 (m, $CH=CH_2$'s ); 5.43 (d, J=14 Hz, $CH_2O$); 5.98 (m, ($CH=CH_2$'s).

IR ($CH_2Cl_2$): 3650, 3530, 1780, 1740$cm^{-1}$

Step B:

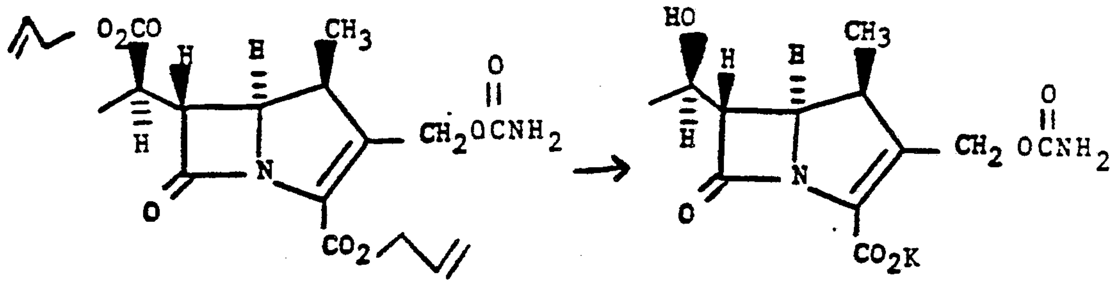

12 13

Carbamate 12 (24 mg, 0.058 mmol) is deblocked as in Example 1-Step F. The reaction mixture is concentrated *in vacuo*, and the residue is washed with diethyl ether (2x1 ml). The residual white solid is dried *in vacuo* and then dissolved in water (0.3 ml) and 5% ethanol-water (0.3 ml). The resulting solution is chromatographed on two 500μ RPS-F plates (eluting with 5% ethanol in water in a cold room). Work-up of the plates, followed by lyophilization, as described in Example 1-Step F, provides the title compound 13 (11 mg, 60%). Structure assignment is consistent with the nmr and ultraviolet spectra:

200MHz nmr ($D_2O$): δ (ppm) 1.13 and 1.27 (two d's, $CH_3CH$ and $CH_3CHO$); 3.26 (m, $H_1$); 3.44 (dd, J=3 and 6 Hz, $H_6$); 4.24 (m, $H_5$ and $CH_3CHO$); 4.72 (part of d, $CH_2O$); 4.80 (HDO); 5.28 (d, J=14 Hz $CH_2O$).

UV ($H_2O$): λ max 267 nm ($NH_2OH$ extinguishable)

## Example 5

Potassium (1S,1′R,5R,6S)-2-methylaminocarbonyl oxymethyl-6-(1′-hydroxyethyl)-1-methylcarbapen-2-em-3-carboxylate

Step A:

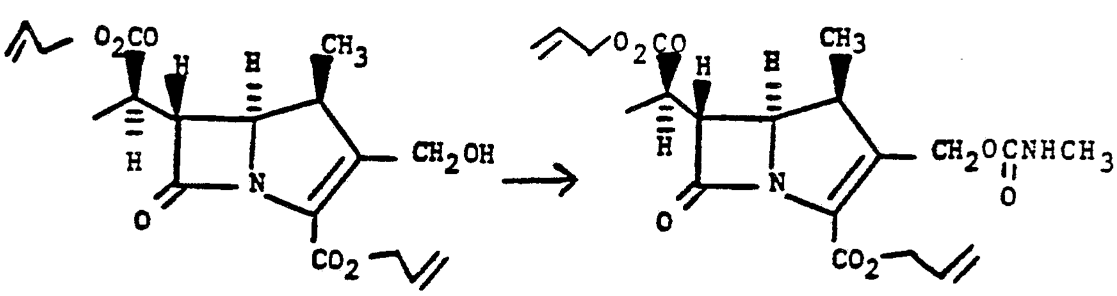

6 14

A solution of 6 (53 mg, 0.15 mmol) in methyisocyanate (650 μl) is heated in a sealed tube at 65°C for 24 hours. The reaction mixture is then cooled and concentrated *in vacuo* to give the crude carbamate 14. Preparative TLC (eluting with 1:1 ethyl acetate-hexane) provides 14 (46 mg, 73 %). Structure assignment is consistent with the nmr and infrared spectra:

200MHz nmr ($CDCl_3$): δ (ppm) 1.18 and 1.45 (two d's, $CH_3CH$ and $CH_3CHO$); 2.81 (d, $NHCH_3$); 3.26 (m,

$H_1$); 3.42 (dd, J=3 and 8 Hz, $H_6$); 4.19 (dd, J=3 and 10 Hz, $H_5$); 4.58-4.86 (m, $CO_2CH_2$'s); 4.86 (d, J=14 Hz, $CH_2O$); 5.12 (m, $CH_3C\underline{H}O$); 5.24-5.50 (m, $CH=C\underline{H}_2$'s); 5.44 (d, J=14 Hz, $CH_2O$); 5.95 (m, $C\underline{H}=CH_2$'s).

IR ($CH_2Cl_2$): 3570, 1780, 1740, 1720cm$^{-1}$

Step B:

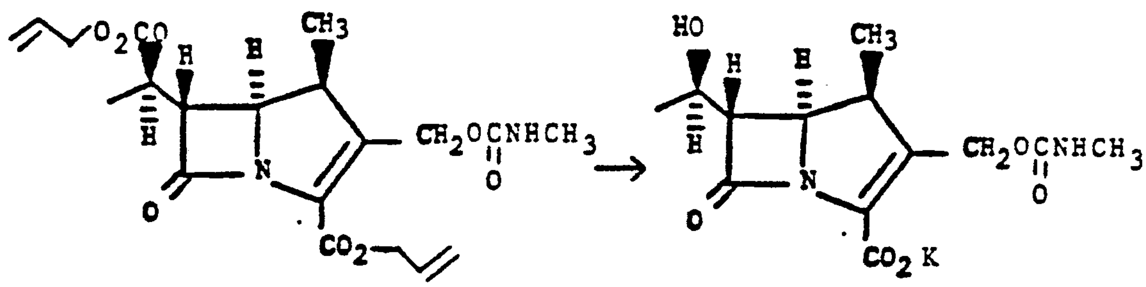

14 15

Compound 14 (58 mg, 0.14 mmol) is deblocked as in Example 1-Step F. The reaction mixture is concentrated in vacuo, and the residue is washed with diethyl ether (2x1.5 ml). The residue is dried in vacuo and then dissolved in water (1 ml). The resulting solution is chromatographed on two 1000µ RPS-F plates (eluting with 5% ethanol-water in a cold room). Work-up of the plates, followed by lyophilization, provides the title compound 15 (30 mg, 64%). Structure assignment is consistent with the nmr and ultraviolet spectra:

200MHz nmr ($D_2O$): δ (ppm) 1.16 and 1.31 (two d's, $C\underline{H}_3CH$ and $C\underline{H}_3CHO$); 2.74 (s, $NHC\underline{H}_3$); 3.27 (m, $H_1$); 3.47 (dd, J=3 and 6 Hz, $H_6$); 4.26 (m, $H_5$ & $CH_3C\underline{H}O$); 4.74 (part of d, $CH_2O$); 4.80 ($H\underline{D}O$); 5.32 (d, J=14 Hz, $CH_2O$).

UV ($H_2O$): λ max 267nm ($NH_2OH$ extinguishable)

## Example 6

Potassium (1S,1'R,5R,6S)-2-phenylaminocarbonyloxymethyl-6-(1'-hydroxyethyl)-1-methylcarbapen-2-em-3-carboxylate

Step A:

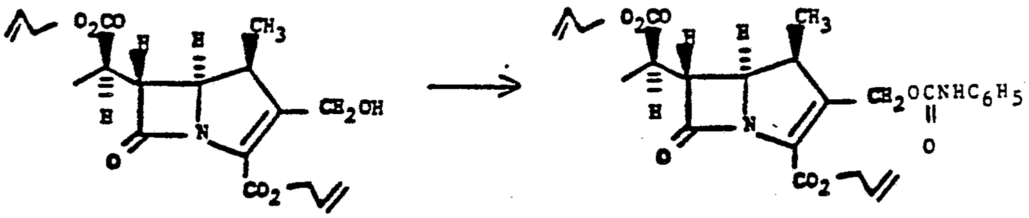

6 16

A solution of 6 (57 mg, 0.16mmol) in phenylisocyanate (300 μl) and toluene (300 μl) is heated in a sealed tube at 70°C overnight. After concentration in vacuo, the residue is purified by preparative TLC (eluting with 10% ethyl acetate-dichloromethane) to give 16 (41 mg, 54%). Structure assignment is consistent with the nmr and infrared spectra:

200MHz nmr ($CDCl_3$): δ (ppm) 1.21 and 1.46 (two d's, $CH_3CH$ and $CH_3CHO$); 3.31 (m, $H_1$); 3.43 (dd, J=3 and 8 Hz, $H_6$); 4.21 (dd, J=3 and 10 Hz, $H_5$); 4.54-4.88 (m, $CO_2CH_2$'s); 4.95 (d, J=14 Hz, $CH_2O$); 5.12 (m, $CH_3CHO$); 5.22-5.40 (m, $CH=CH_2$'s); 5.52 (d, J=14 Hz, $CH_2O$); 5.93 (m, $CH=CH_2$'s); 6.64 (br s, NH); 7.02-7.40 (m, aromatic protons).

IR ($CH_2Cl_2$): 3530, 1780, 1740$cm^{-1}$

Step B:

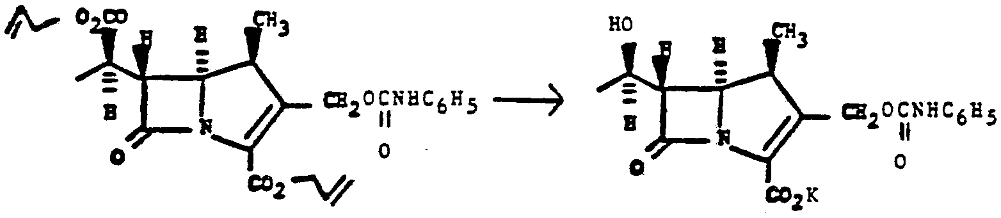

16 17

Compound 16 (40 mg, 0.08 mmol) is deblocked as in Example 1-Step F. The reaction mixture is concentrated in vacuo, and the residue is washed with diethyl ether (2x1.5 ml). The residue is dried in vacuo and then dissolved in dichloromethane (1 ml). The resulting solution is chromatographed on two 1000μ RPS-F plates (eluting with 10% acetonitrile in water in a cold room). Work-up of the plates, followed by lyophilization, as described in Example 1-Step F, provides the title compound 17 (12 mg, 36%). Structure assignment is consistent with the nmr and ultraviolet spectra:

200MHz nmr ($D_2O$): δ (ppm) 1.15 and 1.27 (two d's, $CH_3CH$ and $CH_3CHO$); 3.28 (m, $H_1$); 3.44 (dd, J=3 and 6 Hz, $H_6$); 4.21 (m, $H_5$ and $CH_3CHO$); 4.80 (HDO); 4.87 (part of d, $CH_2O$); 5.39 (d, J=14 Hz, $CH_2O$); 7.11-7.44 (m, aromatic protons).

UV ($H_2O$): λ max 233nm; $NH_2OH$ quenchable at 267nm

Example 7

Potassium (1S,1'R,5R,6S)-2-dimethylamino carbonyloxymethyl-6-(1'-hydroxyethyl)-1-methylcarbapen-2-em-3-carboxylate

Step B:

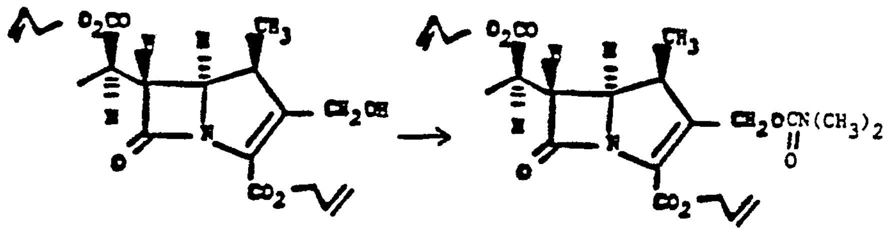

6 18

N,N-Dimethylaniline (13.5 µl, 0.11 mmol) and 1.93 M phosgene in toluene (56 µl, 0.11 mmol) is added to 6 (26 mg, 0.071 mmol) in sieve-dried toluene (1 ml) at 0° under nitrogen. After 1 hour N,N-dimethylaniline (18 µl, 0.15 mmol) and 2.1 M dimethylamine in toluene (68 µl, 0.14 mmol) is added, and the reaction is stirred at 0° for an additional hour. Ethyl acetate (5 ml), 1 M dipotassium hydrogen phosphate (1 ml) and 1 M potassium dihydrogen phosphate (1 ml) is added. After phase separation, the aqueous layer is extracted again with ethyl acetate, and the combined organic layers are washed with brine, dried, filtered, and concentrated *in vacuo* to provide the crude carbamate 18. Preparative TLC (eluting with 30% acetone in hexanes) provides pure 18 (14 mg, 45%). Structure assignment is consistent with the nmr and infrared spectra:

300MHz nmr ($CDCl_3$): δ (ppm) 1.19 and 1.47 (two d's, $C\underline{H}_3CH$ and $C\underline{H}_3CHO$); 2.90 and 2.92 (two s, N-methyls); 3.27 (m, $H_1$); 3.41 (dd, J=3 and 8 Hz, $H_6$); 4.19 (dd, J=3 and 10 Hz, $H_5$); 4.56-4.84 (m, $CO_2CH_2$'s); 4.86 (d, J=14 Hz, $CH_2O$); 5.12 (m, $CH_3C\underline{H}O$); 5.24-5.50 (m, $CH=C\underline{H}_2$'s); 5.42 (d, J=14 Hz, $CH_2O$); 5.95 (m, $C\underline{H}=CH_2$'s).

IR ($CH_2Cl_2$): 1780, 1750, 1700cm$^{-1}$

Step B:

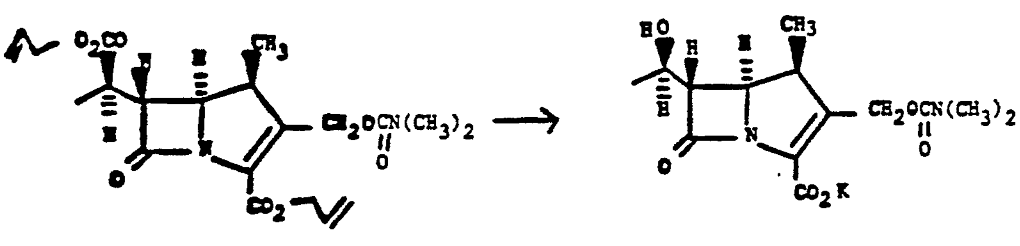

18 19

Compound 18 (27 mg, 0.062 mmol) is deblocked as in Example 1-Step F. The reaction mixture is concentrated *in vacuo* and the residue is washed with diethyl ether (2x1 ml). The residue is dried *in vacuo* and the dissolved in water (0.8 ml). The resulting solution is chromatographed on two 1000µ RPS-F plates (eluting with 12% acetonitrile in water in the cold room). Work-up of the plates followed by lyophilization, as described in Example 1-Step F, provides the title compound 19 (13 mg, 59%). Structure assignment is consistent with the nmr and ultraviolet spectra:

200MHz nmr ($D_2O$): δ (ppm) 1.15 and 1.29 (two d, $C\underline{H}_3CH$ and $C\underline{H}_3CHO$); 2.92 (s, N-methyls); 3.27 (m, $H_1$); 3.44 (dd, J=3 and 6 Hz, $H_6$); 4.21 (m, $H_5$ and $CH_3C\underline{H}O$); 4.72 (part of d, $CH_2O$); 4.80 (HDO); 5.30 (d,

$J = 14$ Hz, $CH_2O$).

UV ($H_2O$): λ max 266nm ($NH_2OH$ quenchable)

## Example 8

(1S,1′R,5R,6S)-6-(1′-hydroxyethyl)-1-methyl-2-(2″-pyridiniumethylaminocarbonyloxymethyl)carbapen-2-em-3-carboxylic acid

Step A:

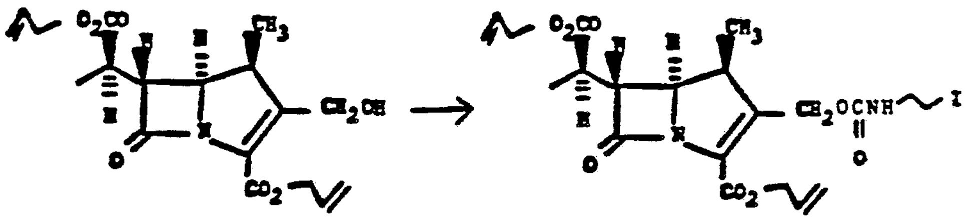

6 → 20

Pyridine (21µl, 0.26 mmol) and iodoethylisocyanate (62 µl, 0.62 mmol) are added to 6 (93 mg, 0.25 mmol) in toluene (1 ml), and the solution is heated at 70° for 2 hours under nitrogen. After concentration in vacuo, preparative TLC (eluting with 10% ethyl acetate in dichloromethane) provides 20 (104 mg, 74%). Structure assignment is consistent with the nmr and infrared spectra and mass spectrometry:

300MHz nmr ($CDCl_3$): δ (ppm) 1.19 and 1.47 (d, $CH_3CH$ and $CH_3CHO$); 3.27 (m, $CH_2I$); 3.42 (dd, $J=3$ and 8 Hz, $H_6$); 3.55 (m, $NCH_2$); 4.20 (dd, $J=3$ and 10 Hz, $H_5$); 4.58-4.86 (m, $CO_2CH_2$'s); 4.86 (d, $J=14$ Hz, $CH_2O$); 5.12 (m, $CH_3CHO$); 5.22-5.48 (m, $CH=CH_2$'s); 5.43 (d, $J=14$ Hz, $CH_2O$); 5.96 (m, $CH=CH_2$'s).

IR ($CH_2Cl_2$): 3520, 1780, 1740(sh), 1725cm$^{-1}$

MS(FAB): 563 (M+1)

Step B:

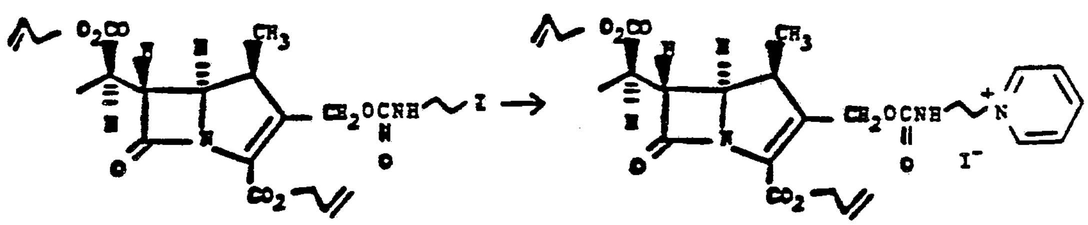

20 → 21

Compound 20 (104 mg, 0.19 mmol) is heated at 70°C for 2 hours under nitrogen in pyridine (1 ml). After concentration in vacuo, preparative TLC (eluting with 10% methanol in dichloromethane) provides pure 21 (62 mg, 52%). Structure assignment is consistent with the nmr and infrared spectra:

300MHz nmr ($CDCl_3$): δ (ppm) 1.14 and 1.46 (two d's, $CH_3CH$ and $CH_3CHO$); 3.33 (m, $H_1$); 3.42 (dd,

J = 3 and 8 Hz, $H_6$); 3.93 (m, $NHCH_2$); 4.20 (dd, J-3 and 10 Hz. $H_5$); 4.59 (d, J = 14 Hz, $CH_2O$); 4.66-4.86 (m, $CO_2CH_2$'s); 5.18 (m, $CH_3CHO$); 5.28-5.52 (m, $CH_2$-pyr, CH = $CH_2$'s, $CH_2O$); 5.96 (m, CH = $CH_2$'s); 6.67 (t, NH); 8.08, 8.52 and 9.28 (t, t, d, aromatic protons).

IR ($CH_2Cl_2$): 1780, 1750, 1725cm$^{-1}$

Step C:

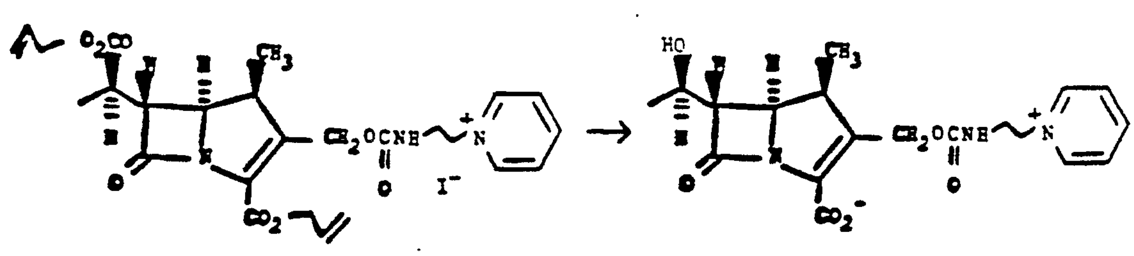

21 22

Compound 21 (62 mg, 0.097 mmol) is deblocked as in Example 1-Step F using triphenylphosphine (15 mg) and tetrakistriphenylphosphine palladium (20 mg) as catalysts. The reaction mixture is concentrated in vacuo and the residue is washed with diethyl ether (2x1 ml). The residue is dried in vacuo and then dissolved in 10% tetrahydrofuran in water (1 ml). The solution is chromatographed on two 1000μ RPS-F plates (eluting with 10% tetrahydrofuran in water in the cold room). Work-up of the plates, followed by lyophilization, as described in Example 1-Step F, provides the title compound 22 (11 mg, 29%). Structure assignment is consistent with the nmr and ultraviolet spectra:

300MHz nmr ($D_2O$): δ (ppm) 1.05 and 1.27 (two d's, $CH_3CH$ and $CH_3CHO$); 3.08 (m, $H_1$); 3.41 (dd, J = 2.5 + 6Hz, $H_6$); 3.72 (m, $NHCH_2$); 4.14 (dd, J = 2.5 & 10 Hz, $H_5$); 4.54 (d, J = 14 Hz, $CH_2O$); 4.70 (m, $CH_2$-pyr); 4.80(HDO); 5.12 (d, J = 14 Hz, $CH_2O$); 8.06, 8.57 and 8.84 (aromatic protons).

UV ($H_2O$): λ max 256nm, shoulder at 263 nm

## Example 9

(1S,1′R,5R,6S)-2-aminomethyl-6-(1′-hydroxyethyl)-1-methylcarbapen-2-em-3-carboxylic acid, Method A

Step A:

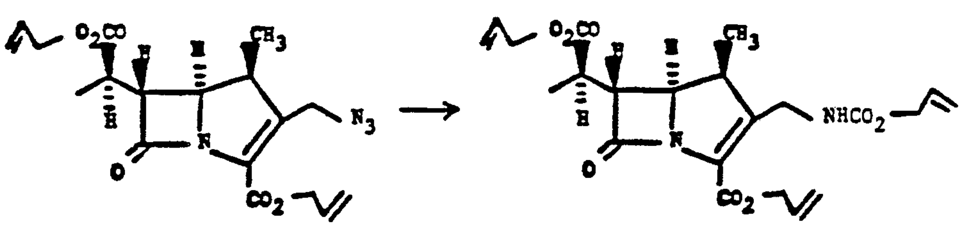

10 23

Triphenylphosphine (91 mg, 0.35 mmol), allyl chloroformate (55 μl. 0.52 mmol) and a solution of sodium bicarbonate (43 mg, 0.51 mmol) in water (1.2 ml) are added to a solution of 10 (97 mg, 0.25 mmol)

dissolved in diethyl ether (48 ml) and dichloromethane (1.2 ml). The reaction mixture is stirred vigorously at ambient temperature under nitrogen for 1 hour. An additional amount of each reagent (amounts as above) is added and stirring is continued for 1.5 hours. After a third addition of each reagent (amounts as above), stirring is continued for 3.5 hours. The reaction mixture is added to 0.5m pH7 phosphate buffer (50 ml) and diethyl ether (30 ml). After phase separation, the aqueous layer is again extracted with diethyl ether, and the combined organic layers are washed with brine, dried, filtered, and concentrated to give 377mg of the crude carbamate 23. Preparative TLC (eluting with 30% ethyl acetate in hexanes) provides purified 23 (34 mg, 31%). Structure assignment is consistent with the nmr and infrared spectra:

200MHz nmr ($CDCl_3$): δ (ppm) 1.20 and 1.46 (two d's, $CH_3CH$ and $CH_3CHO$); 3.22 (m, $H_1$); 3.42 (dd, J=3 and 8 Hz, $H_6$), 4.05 (dd, J=7 and 16 Hz, $CH_2NH$); 4.20 (dd, J=3 and 10 Hz, $H_5$); 4.40 (dd, J=6 and 16 Hz, $CH_2NH$); 4.54-4.90 (m, $CO_2CH_2$'s); 5.15 (m, $CH_3CHO$); 5.26-5.52 (m, $CH=CH_2$'s); 5.95 (m, $CH=CH_2$'s).

IR($CH_2Cl_2$): 3340, 1775, 1750, 1720$cm^{-1}$

Step B:

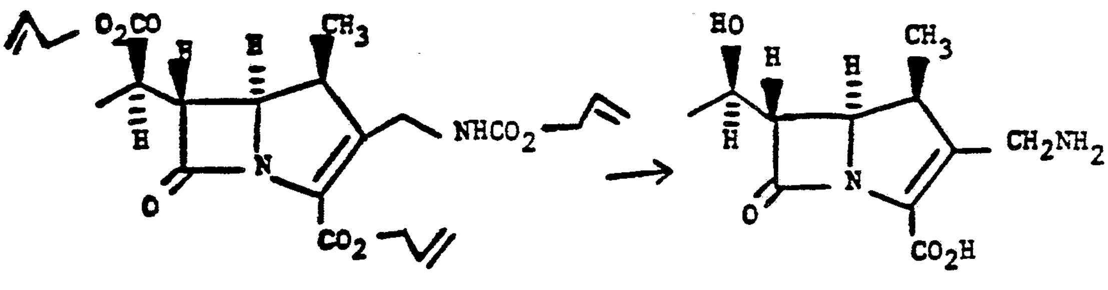

23 24

A mixture of 13 (5.5 mg, 0.012 mmol), triphenyl phosphine (0.8 mg, 0.003 mmol), tetrakistriphenylphosphine (1.4 mg, 0.0012 mmol) 0.5M potassium 2-ethylhexanoate in ethyl acetate (26 µl, 0.013 mmol), and 2-ethylhexanoic acid (4.3 µl, 0.026 mmol) in ethyl acetate (250 µl) and dichloromethane (250 µl) is stirred under nitrogen at ambient temperature for 2 hours. The solution is applied to one 500µ RPS-F gel plate (eluting with 5% ethanol in water in a cold room). Work-up of the plate, followed by lyophilization, as described in Example 1-Step F, provides 3.6 mg of material which by 200MHz nmr is shown to be partially deblocked at the acid and hydroxyl sites but not at the amine. The material is further treated with triphenylphosphine (1.2 mg), tetrakistriphenylphosphine (1.4 mg), and 2-ethylhexanoic acid (5µl) in ethyl acetate (200 µl) and dichloromethane (200 µl), stirred for a few hours at ambient temperature and then allowed to sit overnight in the refrigerator. The reaction mixture is then concentrated in vacuo. The residue is dissolved in 5% ethanol in water (400 µl) and purified on one 500µ RPS-F gel plate (eluting with 5% ethanol in water in a cold room). Work-up of the plate and lyophilization, as above, provides the completely deblocked title compound 24 (500 µg, 15%). Structure assignment is consistent with the nmr and ultraviolet spectra:

200MHz nmr ($D_2O$): δ (ppm) 1.14 and 1.27 (two d's, $CH_3CH$ and $CH_3CHO$); 3.22 (m, $H_1$,); 3.46 (dd, J=3 and 6 Hz, $H_6$); 3.80 (d, J=15 Hz, $CH_2NH_2$); 3.96 (d, J=15 Hz, $CH_2NH_2$); 4.23 (m, $H_5$ and $CH_3CHO$); 4.80 (HDO).

UV ($H_2O$): λ max 269 nm ($NH_2OH$ quenchable)

Example 10

(1S,1′R,5R,6S)-2-aminomethyl-6-(1′-hydroxyethyl)-1-methylcarbapen-2-em-3-carboxylic acid, Method B

Step A:

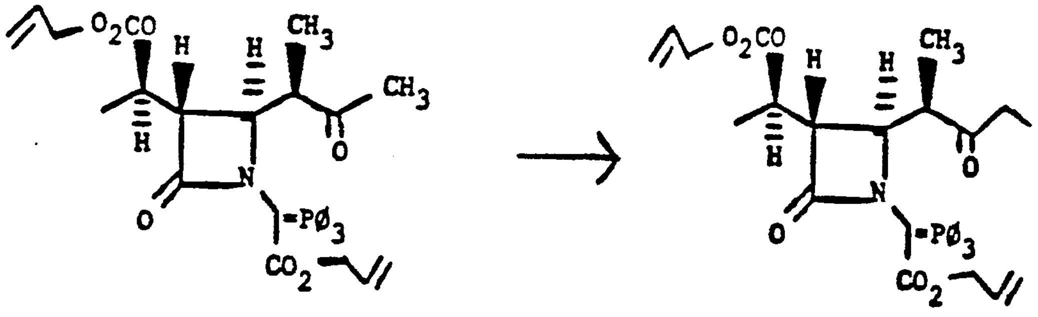

4 25

Lithium diisopropylamide solution (0.19 mmol) prepared as in Example 1, Step D, is added to a solution of 4 (102 mg, 0.16 mmol) in tetrahydrofuran (3 ml) at -78° C. After 30 seconds, the aminating reagent O-(2,4,6-triisopropylbenzenesulfonyl) hydroxylamine (49 mg, 0.16 mmol) is added. The bath temperature is raised to -30° C and stirring under nitrogen is continued for 40 minutes. The reaction mixture is then poured into a stirred mixture of 1M dipotassium hydrogen phosphate (2 ml), 1M potassium dihydrogen phosphate (2 ml), water, and ethyl acetate. The layers are separated and the aqueous layer is extracted again with ethyl acetate. The combined organic layers are extracted with brine, dried over sodium sulfate, filtered, and concentrated in vacuo to give the crude aminomethyl ketone 25. Preparative TLC (eluting with 15% methanol in methylene chloride) affords 25 (54 mg, 52%) as a foam. The material is used immediately in subsequent reactions without further purification.

Step B:

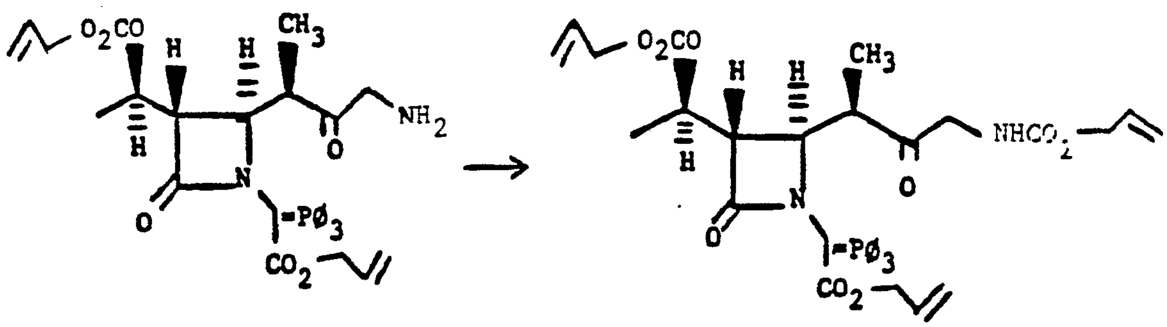

25 26

To a stirred solution of 25 (25 mg, 0.039 mmol) in dichloromethane (1 ml) at 0° C under nitrogen is added 4-dimethylaminopyridine (5.3 mg, 0.043 mmol) and allyl chloroformate (4.5 µl, 0.042 mmol). After a few minutes at 0° C the reaction mixture is stirred at ambient temperature for 30 minutes. Additional dichloromethane is added and the solution is washed with a mixture of 1M dipotassium hydrogen phosphate (1 ml), 1M potassium dihydrogen phosphate (1 ml), and water (2 ml). The layers are separated and the aqueous layer is extracted with dichloromethane. The combined organic layers are washed with brine, dried over sodium sulfate, filtered, and concentrated in vacuo to give the crude carbamate 26. Preparative TLC (eluting with 60% acetone in hexane) provides 26 (15 mg, 54%). Structure assignment is consistent with nmr and infrared spectra and mass spectrometry:

200MHz nmr($CDCl_3$) (selected absorbances): $\delta$ (ppm) 1.08(d, $C\underline{H}_3CHO$); 1.40 (d, $C\underline{H}_3CHC=O$); 5.94 (m, $CH_2C\underline{H}=CH_2$); 7.44-7.88 (aromatic protons)

IR($CH_2Cl_2$): 3430, 1750, 1725, 1660, 1625 $cm^{-1}$

MS(FAB): 727(M+1), 669 (M-Oallyl), 262 ($(C_6H_5)_3P$)

Step C:

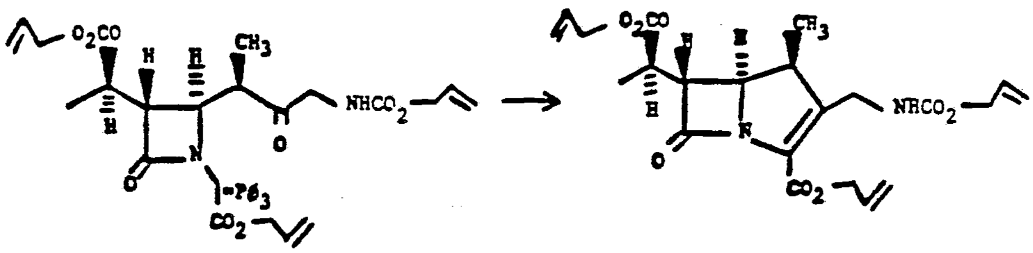

26 23

A solution of 26 (64 mg, 0.088 mmol) in toluene (6 ml) is heated at reflux under nitrogen for 2.5 hours. After concentration in vacuo, the crude material is purified by preparative TLC (eluting with 60% acetone in hexane) to give 23 (37 mg, 95%). Samples of compound 23 prepared in this manner are identical to those prepared as in Example 9, Step A, as determined by nmr and infrared spectroscopy.

Step D:

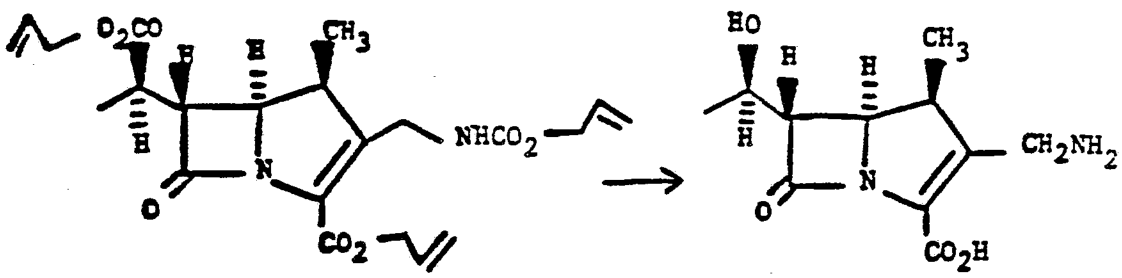

23 24

Tetrakistriphenylphosphine (1.4 mg, 0.0012 mmol) is added to a stirred solution of 23 (10 mg, 0.024 mmol) in tetrahydrofuran (300 µl), followed by the addition of tri-n-butyltin hydride (21 µl, 0.078 mmol). The yellow solution is stirred for two hours at ambient temperature under nitrogen, after which acetic acid (8.2 µl, 0.14 mmol) is added. After the mixture is stirred for forty minutes, hexane (400 µl) is added and the reaction mixture is centrifuged. The supernatant is removed and the insoluble residue is slurried in hexane (1 ml). After centrifugation, the supernatant is again removed and the residue is dried briefly in vacuo. The residue is dissolved in water (400 µl) and the solution is chromatographed on one 500µ RPS-F plate (eluting with 5% ethanol in water in a cold room). Work-up of the plate, followed by lyophilization, as described in Example 1-Step F, provides 24 (2.2 mg, 39%). Samples of compound 24 prepared in this manner are identical to those prepared as in Example 9, Step B, as determined by nmr and ultraviolet spectroscopy.

Example 11

**Table I: Relative Antibacterial Potencies and Dehydropeptidase-I Susceptibilities**

| Organism | Compound (Example No.) 1 | 2 | 3 | 4 | 5 | 6 | 7 | 8 | 9 |
|---|---|---|---|---|---|---|---|---|---|
| S. aureus | 0.4 | 0.7 | 1.4 | 0.9 | 0.8 | 1.2 | 1.4 | 0.87 | 0.6 |
| Enterococcus | 1.0 | 2.8 | 2.0 | 5.7 | 3.7 | 2.8 | 1.2 | 2.3 | 0.3 |
| E. coli | 2.3 | 4.0 | 9.2 | 6.1 | 5.7 | 0.6 | 0.6 | 0.93 | 0.2 |
| Enterobacter | 3.7 | 7.0 | 11.3 | 22.6 | 9.8 | 0.2 | 0.8 | 2.1 | 0.3 |
| Klebsiella | 2.0 | 2.5 | 4.3 | 7.0 | 7.0 | 0.2 | 0.4 | 1.1 | 0.2 |
| Serratia | 4.3 | 12.1 | 22.6 | 32 | 16 | 0.3 | 1.4 | 2.1 | 0.2 |
| Proteus (Ind. + and -) | 3.0 | 27.9 | 29.9 | 14.9 | 16 | 4.9 | 11.3 | 2.5 | 0.2 |
| Ps. aeruginosa | 0.2 | 0.09 | 0.06 | 0.4 | 0.2 | 0.03 | 0.04 | 0.33 | 0.4 |
| DHP-I susceptibility | 0.76 | 0.52 | 7.29 | 0.39 | 0.59 | 1.35 | 0.29 | 0.05 | 0.06 |

Antibacterial potencies were determined using standard agar disc diffusion assays and are reported relative to thienamycin (=1.0). Dehydropeptidase-I (DHP-I) susceptibilities were determined by the method of Kropp et al., Antimicrobial Agents Chemother., 22, 62-70 (1982), and are reported relative to thienamycin (=1.0).

## Claims

1. A compound of the formula:

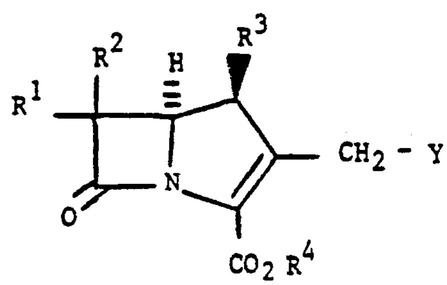

wherein $R^1$ and $R^2$ are independently:

a) hydrogen;
b) $C_1$-$C_4$ alkyl;
c) 1-hydroxy-substituted $C_1$-$C_4$ alkyl; or

d) fluorinated $C_1$-$C_4$ alkyl; with the proviso that at least one of $R^1$ and $R^2$ is hydrogen; $R^3$ is $C_1$-$C_4$ alkyl;

$R^4$ is:

a) hydrogen, or a pharmaceutically acceptable ester or base addition salt thereof; or

b) a removable carboxy protecting group; and Y is:

a) $OR^5$,

wherein $R^5$ is:

i) $C_2$-$C_6$ alkanoyl; or

ii) $-(C=O)NR^8R^9$,

wherein $R^8$ is:

A) hydrogen;

B) $C_1$-$C_4$ alkyl;

C) $C_1$-$C_4$ haloalkyl;

D) phenyl; or

E) $C_1$-$C_4$ alkyl substituted with

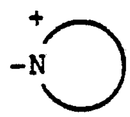 ;

and wherein $R^9$ is:

A) hydrogen; or

B) $C_1$-$C_4$ alkyl; or

wherein $R^8$ and $R^9$ taken together are $C_4$-$C_6$ alkylene;

b) $NR^6R^7$, or a pharmaceutically acceptable acid addition salt, wherein $R^6$ is:

i) hydrogen;

ii) $C_1$-$C_4$ alkyl;

iii) $C_2$-$C_6$ alkanoyl;

iv) $-(C=O)NR^{10}R^{11}$,

wherein $R^{10}$ and $R^{11}$ are independently:

A) hydrogen; or

B) $C_1$-$C_4$ alkyl; or

wherein $R^{10}$ and $R^{11}$ taken together are $C_4$-$C_6$ alkylene; or

v) $-(C=NR^{13})R^{12}$,

wherein $R^{12}$ is:

A) hydrogen;

B) $C_1$-$C_4$ alkyl; or

C) $NR^{14}R^{15}$,

wherein $R^{14}$ and $R^{15}$ are independently:

I) hydrogen; or

II) $C_1$-$C_4$ alkyl; or

wherein $R^{14}$ and $R^{15}$ taken together are $C_4$-$C_6$ alkylene; or

wherein $R^{13}$ and $R^{14}$ taken together are $C_2$-$C_4$ alkylene; and

wherein $R^{13}$ is:

A) hydrogen; or

B) $C_1$-$C_4$ alkyl; and

wherein $R^7$ is:

i) hydrogen; or

ii) $C_1$-$C_4$ alkyl; or

c) $N_3$.

2. A compound according to Claim 1 having the formula:

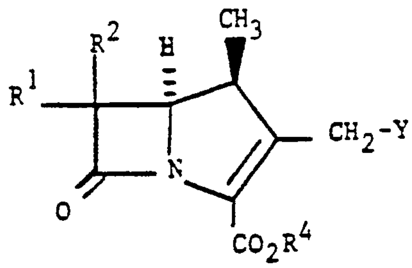

wherein one of $R^1$ or $R^2$ is hydrogen and the other of $R^1$ or $R^2$ is 1-hydroxy-substituted $C_1$-$C_4$ alkyl; $R^4$ is hydrogen, or a pharmaceutically acceptable ester or base addition salt thereof; and
Y is:
a) $OR^5$,
wherein $R^5$ is:
i) $C_2$-$C_6$ alkanoyl; or
ii) $-(C=O)NR^8R^9$,
wherein $R^8$ is:
A) hydrogen;
B) $C_1$-$C_4$ alkyl;
C) $C_1$-$C_4$ haloalkyl;
D) phenyl; or
E) $C_1$-$C_4$ alkyl substituted with

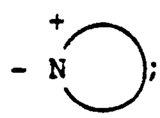

and
wherein $R^9$ is:
A) hydrogen; or
B) $C_1$-$C_4$ alkyl; or
wherein $R^8$ and $R^9$ taken together are $C_4$-$C_6$ alkylene;
b) $NR^6R^7$, or a pharmaceutically acceptable acid addition salt, wherein $R^6$ is:
i) hydrogen; or
ii) $C_1$-$C_4$ alkyl;
iii) $C_2$-$C_6$ alkanoyl;
iv) $-(C=O)NR^{10}R^{11}$,
wherein $R^{10}$ and $R^{11}$ are independently:
A) hydrogen; or
B) $C_1$-$C_4$ alkyl; or
wherein $R^{10}$ and $R^{11}$ taken together are $C_4$-$C_6$ alkylene; or
v) $-(C=NR^{13})R^{12}$,
wherein $R^{12}$ is:
A) hydrogen;
B) $C_1$-$C_4$ alkyl; or
C) $NR^{14}R^{15}$,
wherein $R^{14}$ and $R^{15}$ are independently:
I) hydrogen; or
II) $C_1$-$C_4$ alkyl; or
wherein $R^{14}$ and $R^{15}$ taken together are $C_4$-$C_6$ alkylene; or
wherein $R^{13}$ and $R^{14}$ taken together are $C_2$-$C_4$ alkylene; and
wherein $R^{13}$ is:
A) hydrogen; or
B) $C_1$-$C_4$ alkyl; and

wherein $R^7$ is:
i) hydrogen; or
ii) $C_1$-$C_4$ alkyl; or
c) $N_3$.

3. A compound according to Claim 2 having the formula:

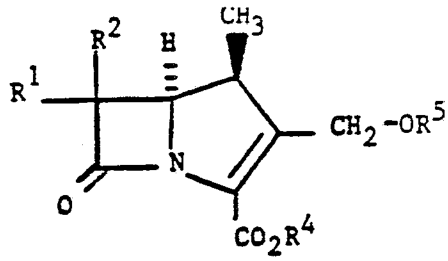

wherein one of $R^1$ or $R^2$ is hydrogen and the other of $R^1$ or $R^2$ is 1-hydroxy-substituted $C_1$-$C_4$ alkyl;
$R^4$ is hydrogen, or a pharmaceutically acceptable ester or base addition salt thereof; and
$R^5$ is:
i) $C_2$-$C_6$ alkanoyl; or
ii) -(C=O)$NR^8R^9$,
wherein $R^8$ is:
A) hydrogen;
B) $C_1$-$C_4$ alkyl;
C) $C_1$-$C_4$ haloalkyl;
D) phenyl; or
E) $C_1$-$C_4$ alkyl substituted with

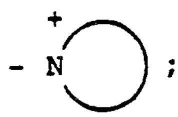

and
wherein $R^9$ is:
A) hydrogen; or
B) $C_1$-$C_4$ alkyl; or
wherein $R^8$ and $R^9$ taken together are $C_4$-$C_6$ alkylene.

4. A compound according to Claim 3 wherein $R^5$ is -(C=O)$NR^8R^9$,
wherein $R^8$ is:
A) hydrogen;
B) $C_1$-$C_4$ alkyl;
C) $C_1$-$C_4$ haloalkyl;
D) phenyl; or
E) $C_1$-$C_4$ alkyl substituted with

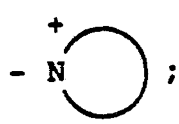

and
wherein $R^9$ is:
A) hydrogen; or
B) $C_1$-$C_4$ alkyl; or
wherein $R^8$ and $R^9$ taken together are $C_4$-$C_6$ alkylene.

5. A compound according to Claim 4 wherein said compound is selected from the group consisting of:
potassium (1S,1′R,5R,6S)-2-aminocarbonyloxymethyl-6-(1′-hydroxyethyl)-1-methylcarbapen-2-em-3-carboxylate,
potassium (1S,1′R,5R,6S)-2-methylaminocarbonyloxymethyl-6-(1′-hydroxyethyl)-1-methylcarbapen-2-em-3-carboxylate,
potassium (1S,1′R,5R,6S)-2-phenylaminocarbonyloxymethyl-6-(1′-hydroxyethyl)-1-methylcarbapen-2-em-3-carboxylate,
potassium (1S,1′R,5R,6S)-2-dimethylaminocarbonyloxymethyl-6-(1′-hydroxyethyl)-1-methylcarbapen-2-em-3-carboxylate, and
(1S,1′R,5R,6S)-6(1′-hydroxyethyl)-1-methyl-2-(2″-pyridiniumethylaminocarbonyloxymethyl)carbapen-2-em-3-carboxylic acid.

6. A compound according to Claim 2 having the formula:

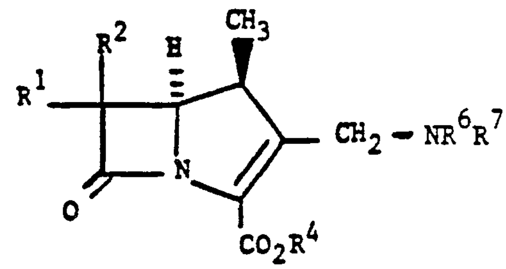

or a pharmaceutically acceptable acid addition salt thereof;
wherein one of $R^1$ or $R^2$ is hydrogen and the other of $R^1$ or $R^2$ is 1-hydroxy-substituted $C_1$-$C_4$ alkyl;
$R^4$ is hydrogen, or a pharmaceutically acceptable ester or base addition salt thereof;
$R^6$ is:
i) hydrogen;
ii) $C_1$-$C_4$ alkyl;
iii) $C_2$-$C_6$ alkanoyl;
iv) $-(C=O)NR^{10}R^{11}$,
where $R^{10}$ and $R^{11}$ are independently:
A) hydrogen; or
B) $C_1$-$C_4$ alkyl; or
wherein $R^{10}$ and $R^{11}$ taken together are $C_4$-$C_6$ alkylene; or
v) $-(C=NR^{13})R^{12}$,
wherein $R^{12}$ is:
A) hydrogen;
B) $C_1$-$C_4$ alkyl; or
C) $NR^{14}R^{15}$,
wherein $R^{14}$ and $R^{15}$ are independently:
I) hydrogen; or
II) $C_1$-$C_4$ alkyl; or
wherein $R^{14}$ and $R^{15}$ taken together are $C_4$-$C_6$ alkylene; and
wherein $R^{13}$ is:
A) hydrogen; or
B) $C_1$-$C_4$ alkyl; and
$R^7$ is:
i) hydrogen; or
ii) $C_1$-$C_4$ alkyl.

7. A pharmaceutical composition useful as an antibacterial agent comprising a pharmaceutically effective amount of at least one compound according to Claim 1, together with one or more non-toxic pharmaceutically acceptable carriers.

8. The use of a compound as claimed in Claim 1 for the preparation of a medicament useful for treating bacterial infections in mammals.

9. The use of a composition as claimed in Claim 7 for the preparation of a medicament useful for treating bacterial infections in mammals.

European Patent Office

# EUROPEAN SEARCH REPORT

Application number: EP 88304286.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BE - A - 905 784 (SHIONOGI)<br>* Claims; examples; table II *<br>-- | 1-5,7-9 | C 07 D 487/04<br>A 61 K 31/40<br>//(C 07 D 487/04,<br>C 07 D 209:00,<br>C 07 D 205:00) |
| D,X | EP - A1 - 0 184 844 (MERCK)<br>* Claims 1 (X=NH), 7 *<br>---- | 1,2,6-9 | |

TECHNICAL FIELDS SEARCHED (Int. Cl.4)

C 07 D 487/00

The present search report has been drawn up for all claims

| Place of search | Date of completion of the search | Examiner |
|---|---|---|
| VIENNA | 25-08-1988 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82